(19) Europäisches Patentamt / European Patent Office / Office européen des brevets

(11) **EP 2 610 617 B1**

(12) **FASCICULE DE BREVET EUROPEEN**

(45) Date de publication et mention de la délivrance du brevet:
**18.02.2015 Bulletin 2015/08**

(51) Int Cl.:
***G01N 33/00*** *(2006.01)*

(21) Numéro de dépôt: **12199524.5**

(22) Date de dépôt: **27.12.2012**

(54) **Dispositif et procédé de détection d'une contamination fongique dans un environnement intérieur**

Vorrichtung and Verfahren zur Erkennung einer Pilzbelastung in Raumlauft

Device and method for the detection of a fungal contamination in ambient air

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.12.2011 FR 1104126**

(43) Date de publication de la demande:
**03.07.2013 Bulletin 2013/27**

(73) Titulaire: **Centre Scientifique et Technique du Batiment**
**77447 Marne La Vallée Cedex 2 (FR)**

(72) Inventeurs:
• **Moularat, Stéphane**
 **77185 LOGNES (FR)**
• **Joblin, Yaël**
 **94450 Lemeil-Brevannes (FR)**
• **Robine, Enric**
 **77400 LAGNY SUR MARNE (FR)**

(74) Mandataire: **Chaffraix, Jean**
**Cabinet Blétry & Associés**
**82 Boulevard de Sébastopol**
**75003 Paris (FR)**

(56) Documents cités:
**WO-A1-2008/125770 WO-A2-99/47905**

• **Yaël Joblin: "Elaboration d'un microsystème d'analyse de l'air destiné à la détection rapide d'un développement fongique dans les espaces clos", , 12 mai 2011 (2011-05-12), XP55051669, Paris Extrait de l'Internet: URL:http://tel.archives-ouvertes.fr/docs/0 0/69/66/18/PDF/TH2011PEST1027_complete.pdf [extrait le 2013-01-30]**
• **ENRICO COZZANI: "MODELING, DESIGN AND EXPERIMENTAL CHARACTERIZATION OF MICRO-ELECTRO-MECHANICAL SYSTEMS FOR GAS-CHROMATOGRAPHIC APPLICATIONS", ALMA MATER STUDIORUM UNIVERSITÀ DI BOLOGNA,, 6 mars 2011 (2011-03-06), pages 1-125, XP007920889,**
• **ANNA CAMPAGNOLI ET AL: "Use of the Electronic Nose as a Screening Tool for the Recognition of Durum Wheat Naturally Contaminated by Deoxynivalenol: A Preliminary Approach", SENSORS, vol. 11, no. 5, 1 janvier 2011 (2011-01-01), pages 4899-4916, XP055034128, ISSN: 1424-8220, DOI: 10.3390/s110504899**

**Description**

**DOMAINE TECHNIQUE DE L'INVENTION**

La présente invention concerne un dispositif de détection d'une contamination fongique dans un environnement intérieur, son utilisation ainsi qu'un procédé de détection d'une contamination fongique dans un environnement intérieur mettant en oeuvre un tel dispositif.

L'individu des sociétés industrialisées passe plus de 90 % de son temps dans des espaces clos où il se trouve exposé à de multiples polluants de natures physique, chimique et biologique. Conscients des risques sanitaires potentiellement induits par cette pollution complexe, les pouvoirs publics ont introduit, dans le code de l'environnement du Grenelle 2, le principe de surveillance de la qualité de l'air des environnements intérieurs. Ce principe comprend la mise en place de systèmes de mesure et d'information dans les établissements accueillant des personnes vulnérables (enfants, personnes âgées...), et dans les lieux recevant du public (écoles, transports, musées ....) Ainsi, comme le rappelle l'Organisation Mondiale de la Santé (OMS) dans son rapport paru en 2009 « WHO guidelines for indoor air quality : dampness and mould », les moisissures sont susceptibles d'induire des allergies, des infections, des toxi-infections ou encore des irritations. Outre leur impact sanitaire, ces microorganismes peuvent agir sur la structure même des bâtiments, altérant irrémédiablement produits de construction et de décoration, phénomène largement redouté notamment par les conservateurs du patrimoine.

L'intérêt pour ces microorganismes est exacerbé par le constat que de nombreux logements des pays industrialisés sont concernés par des problèmes d'humidité et/ou de moisissures. Ainsi, des études basées sur des questionnaires ou inspections visuelles montrent une proportion très importante de logements contaminés. Des études européennes rapportent ainsi que la proportion de logements présentant des moisissures visibles peut atteindre 25 % (Brunekreef, 1992; Pirhonen, 1996). Des études réalisées en Amérique du Nord font, quant à elles, état de taux de contamination variant entre 14 et 38 % (Dales, 1991). Cette proportion atteint 80 % lorsque sont prises en compte les habitations avec une forte humidité détectée dans les murs (Miller, 1988; Koskinen, 1999).

En France, les pouvoirs publics subventionnent un Observatoire de la Qualité de l'Air intérieur (OQAI) piloté par le Centre Scientifique et Technique du Bâtiment (CSTB). Les résultats de la campagne nationale logements réalisée par l'OQAI, publiés fin 2007, dressent le premier état de la qualité de l'air des habitations françaises. Cette campagne de mesures révèle notamment que la contamination des logements par les moisissures concerne une proportion importante des foyers français avec des valeurs comprises entre 37 et 42 % dont 2 % (soit plus de 610 000 logements) présentaient des surfaces contaminées de plus de 1 m$^2$ (Moularat, 2008).

Parmi les biocontaminants de ces environnements, les micromycètes (moisissures) constituent un axe de recherche pour de nombreuses équipes à travers le monde (Amérique du Nord, pays d'Europe du Nord, Belgique, Italie, Australie, France...).

Par environnement intérieur on entend un espace confiné à l'intérieur d'un bâtiment qui est aéré de façon non continue. Des exemples d'environnements intérieurs peuvent être trouvés dans les habitations, les musées, les églises, les caves, les monuments historiques, les bâtiments administratifs, les écoles et les hôpitaux.

La présence de moisissures dans les environnements intérieurs n'est pas sans conséquences sanitaires. En effet, de nombreuses études ont démontré l'apparition de symptômes chez les occupants de locaux comportant des moisissures, et également leur rôle dans la dégradation à la fois des matériaux et des ouvrages qu'elles colonisent. En effet, les enzymes et/ou les acides produits par les champignons provoquent également la détérioration de leur support.

Les champignons émettent dès le début de leur développement des molécules volatiles (Composés Organiques Volatils, COV) issues soit de leur métabolisme, soit de la dégradation du matériau sur lequel ils se développent par les enzymes ou les acides qu'ils produisent. Les COV diffusent à travers les parois et peuvent être détectés dans l'air même dans le cas de contaminations cachées. Cependant, les COV présents dans un environnement intérieur peuvent également provenir d'autres sources telles que les matériaux de construction, les produits ménagers ou encore l'activité humaine. Les concentrations des COV d'origine fongique, notamment à un stade précoce de contamination, s'avèrent être relativement faibles comparées à l'ensemble des COV présents dans un environnement intérieur.

ETAT DE LA TECHNIQUE ANTERIEURE

Dans ce contexte, la demanderesse a engagé depuis plus de 10 ans diverses actions de recherche notamment sur la maîtrise du développement de moisissures sur les supports et sur la détection précoce de leur croissance.

Traditionnellement, la contamination fongique d'un environnement est objectivée par son examen visuel ou par la culture des microorganismes présents dans l'air, sur les surfaces ou dans les poussières. De ce fait, les méthodes usuelles permettent rarement de détecter les contaminations cachées (croissance derrière une cloison, dans la structure du bâti ou dans des systèmes de ventilation par exemple) ou récentes pour lesquelles aucun signe de développement n'est apparent.

Dans une optique de détection précoce d'un développement fongique, les travaux de la demanderesse s'appuient sur l'émission, dès les premières heures du développement fongique, de Composés Organiques Volatils d'origine microbienne (COVm) spécifiques, qui diffusent dans l'environnement et constituent une empreinte biochimique dont la détection signe une activité fongique.

Ainsi, afin de détecter l'ensemble des cas de contaminations, une technique basée sur l'identification des traceurs chimiques composant cette empreinte et permettant le calcul d'un indice de contamination a été développée par la demanderesse dans la demande de brevet FR 2913501.

La demande de brevet FR 2913501 propose un procédé de détection d'une contamination fongique dans un environnement intérieur par la détermination d'un indice de contamination fongique basée sur l'analyse des COV présents dans l'air ambiant. Ce procédé permet la détection d'un développement fongique à un stade précoce de son développement même en cas de contamination cachée mais met en oeuvre des méthodes classiques d'analyse, comme la chromatographie en phase gazeuse couplée à un spectromètre de masse. Ces méthodes nécessitent la collecte d'un échantillon qui doit être rapporté au laboratoire où il subira de longues étapes de concentration, de séparation et d'analyse. Ces étapes pour la détection d'une contamination fongique dans un environnement intérieur nécessitent l'intervention d'un technicien qualifié et se révèlent relativement longues et coûteuses. Ces techniques d'analyse ne permettent donc pas une mesure rapide et en continu.

Enrico Cozzani décrit dans « Modeling, design and experimental characterization of micro-electro-mechanical systems for gas-chromatographic applications » (urn:nbn:it:unibo-2609) un module pour identifier des COV, en particulier le benzène, le toluène éthylebenzène et xylène, comprenant un système d'injection, un système de séparation au moyen d'une colonne de chromatographie gazeuse et d'un système de détection.

Les solutions disponibles à ce jour ne permettent donc pas de répondre à la demande de détection précoce et de surveillance en continu des contaminations fongiques. Le principe général du microsystème selon l'invention est décrit dans la demande de brevet N° 10 59636.

La société demanderesse a quant à elle réussi à mettre au point un dispositif de détection d'une contamination fongique dans un environnement intérieur permettant une analyse rapide in situ de l'air ambiant avec un faible temps de mesure, et donc la détection de contamination en continu. Le dispositif de l'invention présente de plus l'avantage de pouvoir être utilisé sans l'intervention d'un technicien spécialisé.

## EXPOSE DE L'INVENTION

Ainsi, la présente invention concerne un dispositif de détection d'une contamination fongique dans un environnement intérieur comprenant :

- un module de préconcentration ;
- un module de séparation comprenant une micro-colonne chromatographique; et
- un module de détection comprenant une matrice de capteurs.

La demanderesse a donc développé un système autonome de microcapteurs chimiques adapté à la mesure in situ. Outre le gain de temps engendré par l'absence de la phase d'analyse en laboratoire, intégrée dans le système à développer, ce dispositif doit assurer une surveillance continue d'environnements fréquentés par le public, tels que les musées, les écoles, les centres hospitaliers....

En particulier, le module de préconcentration se trouve en amont des autres modules du dispositif. L'utilisation d'un préconcentrateur est requise pour l'utilisation de systèmes chromatographiques lorsque la résolution de la colonne chromatographique est trop faible ou que la sensibilité des détecteurs utilisés est limitée par des faibles concentrations des molécules cibles.

La préconcentration se base sur le principe d'accumulation. Lors de l'utilisation d'un préconcentrateur, le flux à analyser, Notamment le gaz, traverse le module de préconcentration et les molécules cibles y sont accumulées, pendant une phase de collecte, sur un matériau adsorbant. Bien entendu, le choix du matériau adsorbant dépend des molécules cibles recherchées de sorte qu'elles peuvent être piégées sur le matériau puis désorbées par exemple thermiquement et injectées dans une colonne chromatographique pour être séparées puis analysées. Ainsi, les molécules cibles libérées permettent d'obtenir, en sortie de colonne, des pics de désorption avec une concentration plus élevée de molécules cibles. Ce module de préconcentration augmente donc l'efficacité de séparation de la colonne et les pics de forte concentration augmentent la sensibilité de l'analyse. Dans l'interprétation de la présente demande, les expressions « module de préconcentration » et « module de concentration » doivent être considérées comme des synonymes.

De préférence, le module de concentration comprend une microstructure de préconcentration. Ce type de microstructure permet d'une part de réaliser un dispositif de plus petite taille, de préférence portable et facile à manipuler. D'autre part, Ce type de microstructure permet non seulement une consommation d'énergie énergétique faible lors de la désorption mais aussi une meilleure efficacité de chauffe liée à une plus faible masse thermique et des volumes morts moins importants.

La présence ou l'absence de moisissure dans un environnement intérieur ne peut pas être déduite à partir de la détection d'un seul COV d'origine fongique. Les présents inventeurs ont donc conçu un dispositif qui utilise un principe de détection d'une contamination fongique reposant sur la détection de certains COV cibles. Le dispositif de l'invention permet donc en particulier la détection de la présence d'une palette de COV cibles pouvant résulter du développement d'une contamination fongique. Les COV cibles comprennent notamment :

(1) les COV qui sont émis indépendamment de l'espèce fongique et de leur support et qui ne sont émis que par des espèces fongiques, tels que le 1-octèn-3-ol, le 1,3-octadiène et le méthyl-2-éthylhexanoate ;

(2) les COV qui sont émis indépendamment de l'espèce fongique et du support, mais qui peuvent également avoir d'autres origines biologiques, tels que le 2-méthylfurane, le 3-méthylfurane, le 3-méthyl-1-butanol, le 2-méthyl-1-butanol et l'$\alpha$-pinène ;

(3) les COV qui sont émis en fonction de l'espèce fongique et/ou du support, tels que le 2-heptène, le diméthylsulfure, le 4-heptanone, le 2(5H)-furanone, le 3-heptanol et le méthoxybenzène.

Les COV cibles peuvent également comprendre des COV n'appartenant pas aux catégories (1), (2) ou (3) mais intervenant dans l'appréciation de la présence d'une contamination fongique, tel que le 2-éthylhexanol.

En particulier, le module de préconcentration du dispositif selon l'invention permet une concentration des COV cibles présents dans l'air ambiant jusqu'à une concentration détectable par le module de détection. La concentration des COV peut être réalisée par toute méthode connue de l'homme du métier, en particulier l'accumulation sur un matériau adsorbant. Le module de préconcentration comprend donc avantageusement un matériau adsorbant permettant l'accumulation des COV cibles. La structure du matériau adsorbant possède typiquement une forme permettant d'optimiser sa surface spécifique. De préférence, le matériau adsorbant est sous forme de particules ayant typiquement une taille de 50 à 200 $\mu$m, une surface spécifique de 20 à 50 m$^2$/g, une porosité de 1 à 5 cm$^3$/g et une taille de pores moyenne de 50 à 500 nm. Le matériau adsorbant est préférentiellement choisi parmi le charbon actif, le gel de silice, les zéolithes et les résines synthétiques poreuses, tels ceux commercialisés sous la marque Tenax®, Carbograph® ou Chromosorb®. Le module de préconcentration comprend avantageusement en outre un système de chauffage permettant la désorption des COV adsorbés sur le matériau adsorbant.

En particulier, lesdits modules sont successivement en aval l'un de l'autre. Selon un premier aspect, le dispositif selon l'invention comprend un moyen de génération de flux de préférence une pompe et au moins une première électrovanne en amont du module de détection permettant soit de diriger un flux qui comprend des molécules cibles vers le module de détection, soit de diriger un flux filtré par un premier moyen de filtration permettant un nettoyage du module de détection lorsque le flux ne comprend pas les molécules cibles. De préférence, le flux filtré est dirigé directement vers le module de détection.

Avantageusement, un même flux est soit dirigé vers le module de détection lorsqu'il comprend des molécules cibles, soit dirigé vers le premier moyen de filtration lorsqu'il ne comprend pas les molécules cibles.

La détermination de la présence ou de l'absence des molécules cibles en particulier de COV cibles, se fait de préférence selon les temps de rétention du module de séparation desdites molécules cibles. Ces temps de rétentions peuvent être estimés par des étalons. Avantageusement, ladite première électrovanne est placée entre le module de séparation et le module de détection.

Selon un autre aspect, le dispositif comprend en outre au moins une deuxième électrovanne en amont du module de séparation permettant de diriger un flux soit vers le module de séparation lorsque le flux comprend des molécules cibles ou lorsque le flux est filtré par un moyen de filtration, soit vers l'extérieur lorsque le flux ne comprend pas les molécules cibles. Avantageusement, ladite deuxième électrovanne est placée entre le module de concentration et le module de séparation.

Ainsi, pendant la rétention des molécules cibles par exemple dans le module de concentration, le module de séparation n'est pas encombré par les autres molécules du prélèvement. En outre, il est possible de faire passer un flux filtré successivement à travers le module de concentration et le module de séparation.

De préférence, le dispositif comprend en outre au moins une troisième électrovanne en amont du module de concentration permettant soit de diriger un flux de prélèvement d'échantillon vers le module de concentration, soit de diriger un flux filtré par un moyen de filtration permettant de nettoyer au moins le module de concentration. Le flux filtré sert de gaz vecteur durant l'analyse.

Ainsi, en dehors des phases de prélèvement, le dispositif peut être nettoyé par un flux filtré.

De préférence, un même moyen de filtration est prévu pour générer un flux d'air filtré pour le nettoyage du module de concentration et du module de séparation.

Selon un aspect avantageux, le premier et/ou le deuxième moyen de filtration comprend un polymère adsorbant. En particulier, le polymère adsorbant des premier et/ou deuxième moyen de filtration est apte à adsorber des molécules volatiles ou semi-volatiles. Ainsi, le passage par un tel moyen de filtration permet de diminuer le bruit de fond lors de l'analyse des molécules cibles et un nettoyage des différents modules. Par exemple, un tel matériau adsorbant comprend une résine polymère poreuse à base d'oxyde de 2.6-diphénylène. Un moyen de filtration comprenant du charbon actif peut également être envisagé.

Avantageusement, les modules de concentration et/ou de séparation comprennent un matériau susceptible d'adsorber ou absorber lesdites molécules cibles associé à un moyen de désorption correspondant. De préférence, le matériau susceptible d'adsorber ou absorber lesdites molécules cibles est un polymère adsorbant tel que le 2,6-diphénylène de préférence des grains de polymère dans le cas du module de concentration et un gel de diméthylpolysiloxane (PDMS) dans le cas du module de séparation, et le moyen de désorption comprend des résistances chauffantes prévues sur lesdits modules de concentration et/ou de séparation.

Selon un autre aspect intéressant, le dispositif comprend en outre une carte de commande permettant de commander de préférence automatiquement, au moins l'un parmi lesdites électrovannes, le moyen d'élution en particulier les résistances chauffantes, le moyen de génération de flux en particulier au moins une pompe.

De préférence, la carte de commande est connectée au module de détection de sorte à recevoir des données de celui-ci. Les cartes de traitement des signaux du module de détection et de commande du système peuvent également être dissociées.

Avantageusement, la carte de commande et le module de détection sont configurés pour mesurer une différence de résistivité entre le flux qui comprend les molécules cibles et le flux filtré. En particulier, le module de détection comprend un montage dit « pont de Wheatstone » associé à un montage d'amplification.

Un autre objet de l'invention consiste en une carte de commande pour un dispositif de détection tel que décrit précédemment, configurée pour commander de préférence automatiquement, lesdites électrovannes, de sorte à réaliser au moins l'un parmi :

- soit diriger un flux qui comprend des molécules cibles vers le module de détection, soit de diriger un flux filtré par un premier moyen de filtration permettant un nettoyage du module de détection lorsque le flux ne comprend pas les molécules cibles,
- diriger un flux soit vers le module de séparation lorsque le flux comprend des molécules cibles ou lorsque le flux est filtré par un deuxième moyen de filtration, soit vers l'extérieur lorsque le flux ne comprend pas les molécules cibles,
- soit de diriger un flux de prélèvement d'échantillon vers le module de concentration, soit de diriger un flux filtré par un troisième moyen de filtration permettant un nettoyage du module de concentration lorsque le flux ne comprend pas les molécules cibles.

De préférence, la carte de commande est configurée pour commander en outre de préférence automatiquement, les moyens de génération de flux en particulier au moins une pompe.

Avantageusement, la carte de commande est configurée pour commander en outre, de préférence automatiquement, le moyen d'élution en particulier les résistances chauffantes de sorte à désorber les molécules cibles.

L'invention porte également sur un procédé de détection d'une contamination fongique dans un environnement intérieur à partir d'un dispositif de détection comprenant :

- un module de préconcentration ;
- un module de séparation comprenant une micro-colonne chromatographique en aval du module de préconcentration ; et
- un module de détection comprenant une matrice de capteurs en aval du module de séparation,
- des moyens de génération de flux de préférence au moins une pompe,

le procédé comprenant des étapes de :

- concentration dans laquelle des molécules cibles sont retenues dans le module de préconcentration de préférence pendant un temps de concentration;
- nettoyage de capteurs, dans laquelle un flux filtré passe à travers au moins l'un parmi le module de préconcentration, le module de séparation et le module de détection,
- analyse, dans laquelle les molécules cibles passent dans le module de détection, de préférence pendant un temps d'analyse.

De manière plus générale, l'invention porte sur un procédé de détection d'une contamination fongique dans un environnement intérieur à partir d'un dispositif de détection comprenant :

- un module de préconcentration ;
- un module de séparation comprenant une micro-colonne chromatographique en aval du module de préconcentration ; et

- un module de détection comprenant une matrice de capteurs en aval du module de séparation,
- des moyens de génération de flux de préférence au moins une pompe,

le procédé comprenant au moins une étape de :

- nettoyage de capteurs, dans laquelle un flux filtré passe à travers au moins l'un parmi le module de préconcentration, le module de séparation et le module de détection.

Selon un aspect avantageux, le procédé comprend au moins une étape inactive (12,13,10) avant et/ou après lesdites étapes de concentration et d'analyse, dans laquelle au moins les moyens de génération de flux sont inactivés, les étapes du procédé étant de préférence mises en oeuvres en continu de sorte à détecter une contamination fongique dans un environnement intérieur.

De préférence, le procédé de détection comprend les étapes pour commander de préférence automatiquement, au moins une électrovannes, de sorte à réaliser au moins l'un parmi :

- soit diriger un flux qui comprend des molécules cibles vers le module de détection, soit de diriger un flux filtré par un premier moyen de filtration permettant un nettoyage du module de détection lorsque le flux ne comprend pas les molécules cibles,
- diriger un flux soit vers le module de séparation lorsque le flux comprend des molécules cibles ou lorsque le flux est filtré par un deuxième moyen de filtration, soit vers l'extérieur lorsque le flux ne comprend pas les molécules cibles,
- soit de diriger un flux de prélèvement d'échantillon vers le module de concentration, soit de diriger un flux filtré par un troisième moyen de filtration permettant un nettoyage du module de concentration lorsque le flux ne comprend pas les molécules cibles.

De préférence, le procédé de détection comprend en outre des étapes pour commander de préférence automatiquement, le moyen de génération de flux en particulier la au moins une pompe de sorte à réaliser lesdites directions de flux.

Avantageusement, le procédé de détection comprend en outre des étapes pour commander de préférence automatiquement, le moyen d'élution en particulier les résistances chauffantes de sorte à désorber les molécules cibles.

L'invention porte également sur un produit de programme informatique chargeable dans la mémoire d'une unité de commande comprenant des parties de code logiciel pour réaliser le procédé de détection selon l'invention lorsqu'il est mis en oeuvre par une unité de commande. Ainsi, la carte de commande décrite précédemment peut par exemple comprendre un tel produit de programme informatique.

Le « collage » de couches diélectriques s'entend par exemple comme une des techniques de « collage » permettant d'obtenir des cavités closes, exposées dans le livre de S. Mir, Charlot (Charlot, 2002). En particulier, le « collage » entre plaques (« *wafer bonding* ») est une technique qui permet de souder ensemble des substrats de silicium ou de matériaux différents (tel que le verre) afin d'obtenir des structures 3D pouvant former des cavités closes. Les deux techniques connues de l'homme du métier sont par exemple la soudure anodique et la soudure par fusion.

Selon un aspect avantageux, le dispositif selon l'invention comprend des capteurs polymères. En effet, les capteurs chimiques sont utilisés pour la mesure en continu des polluants organiques. Cependant, de tels capteurs ne sont pas suffisamment sensibles pour détecter les niveaux de concentrations en COV émis lors d'un développement fongique, ni suffisamment sélectifs pour différencier ces COV d'origine fongique des autres COV provenant d'autres sources tel que les matériaux de construction ou de décoration par exemple.

Préférentiellement, le module de détection comprend un polymère conducteur sélectionné dans le groupe comprenant le PEDOT-PSS, le bifluorène dibromé, le polypyrrole dopé avec de l'octane sulfonate, le polypyrrole dopé avec du perchlorate de lithium ou tout autre dérivé du polypyrrole, du polythiophène ou de la polyaniline.

Selon une variante, dans le dispositif de l'invention, le module de préconcentration comprend un micro-préconcentrateur. Un tel micro-préconcentrateur présente avantageusement un volume utile de 0,1 à 1 cm$^3$, de préférence 0,1 à 0,5 cm$^3$, plus préférentiellement de 0,1 à 0,3 cm$^3$. Le micro-préconcentrateur est constitué d'une plaque de substrat, telle qu'une plaque de silicium, sur la surface duquel sont gravés des sillons dans lesquels se trouve le matériau adsorbant. Une seconde plaque, d'un matériau identique ou différent du substrat (tel qu'une plaque de verre), collée sur la surface de la plaque de substrat gravée comportant les sillons, referme le micro-préconcentrateur. La plaque de substrat a par exemple une surface de 2 à 20 cm$^2$. Les sillons ont avantageusement une longueur de 3 à 10 cm, une largeur de 100 à 1000 $\mu$m, une profondeur de 100 à 500 $\mu$m, et une section de 0,01 à 0,5 mm$^2$. La section des sillons peut présenter diverses formes telles que rectangulaire, semi-circulaire ou circulaire.

Avantageusement, le module de préconcentration comprend également un système de circulation forcée permettant de forcer le passage de l'air ambiant à travers le module de préconcentration.

Le module de séparation comprend une micro-colonne chromatographique ayant avantageusement une section de 0,01 à 0,25 mm$^2$. La longueur de la micro-colonne doit être également choisie de sorte à optimiser la séparation des COV. Elle est avantageusement supérieure à 1 m, de préférence comprise entre 1 et 50 m. Le choix d'une longueur importante permet d'améliorer l'efficacité de la colonne et donc d'obtenir une meilleure séparation des COV. La micro-colonne comprend une phase stationnaire que l'homme du métier saura choisir de sorte à optimiser la séparation des COV. Celle-ci appartient avantageusement à la famille des polysiloxanes (par exemple le diméthylpolysiloxane (PDMS)). Différentes phases stationnaires peuvent également être utilisées. Ces phases peuvent être des hydrocarbures ramifiés, des polyéthylène glycols et polypropylène glycols, des polyesters, des sulfones de polyaryléthers, ou encore des phases stationnaires à sélectivités spécifiques.

La micro-colonne comprend par exemple une plaque de substrat, tel qu'une plaque de silicium, sur la surface duquel est gravé un sillon dans lequel se trouve la phase stationnaire. Une seconde plaque, d'un matériau identique ou différent du substrat (telle qu'une plaque de verre), collée sur la surface de la plaque de substrat gravée comportant le sillon, referme la micro-colonne. La plaque de substrat a typiquement une surface de 2 à 20 cm$^2$. Le sillon a avantageusement une longueur de plus de 1 m, de préférence de 1 à 50 m, une largeur de 100 à 500 $\mu$m, une profondeur de 100 à 500 $\mu$m, et une section de 0,01 à 0,25 mm$^2$. La section des sillons peut présenter diverses formes telles que rectangulaire, semi-circulaire ou circulaire. Le sillon peut être agencé de différentes manières de sorte à minimiser l'encombrement et donc la taille de la structure, par exemple en lacets parallèles (serpentin).

Selon un autre mode de réalisation du dispositif de l'invention, le module de séparation comprend également un système de sélection des COV cibles comprenant de préférence une électrovanne et une unité programmable permettant le contrôle de ladite électrovanne. Ce système de sélection est directement connecté à la sortie de la micro-colonne. Le temps de rétention, pour une phase stationnaire et une longueur de micro-colonne données, est spécifique pour chaque COV. Ainsi, en renseignant les temps de rétention de chaque COV cible, l'unité programmable peut être préprogrammée de sorte que le système de sélection dirige sélectivement les portions d'éluât correspondantes aux temps de rétention de chaque COV cible vers le module de détection, le reste de l'éluât étant évacué du circuit d'analyse. Lesdites portions d'éluât peuvent être soit envoyées les unes après les autres au module de détection, au fur et à mesure de l'élution, soit stockées puis envoyées ensemble dans le module de détection.

Les COV cibles, comprenant principalement des COV d'origine fongique, ont des concentrations très faibles comparées aux concentrations totales de l'ensemble des COV présents dans l'air ambiant. Ainsi, cette séparation sélective des COV cibles permet d'empêcher la formation d'un bruit de fond et/ou les phénomènes d'hystérésis et/ou de saturation des capteurs du module de détection qui seraient préjudiciables à la détection des COV cibles.

Le module de détection du dispositif selon l'invention comprend une matrice de capteurs avantageusement choisis parmi les capteurs électrochimiques de type polymère. Les capteurs comprennent de préférence une couche de polymère ou de mélange de polymères ayant une affinité avec les COV d'origine fongique.

Les COV peuvent être classés en différentes familles selon leur nature chimique : les COV aliphatiques, les alcools, les cétones, les esters, les éthers, les aldéhydes, les COV aromatiques, les COV chlorés, les COV azotés ou les COV soufrés. Il existe des capteurs chimiques permettant la détection de composés possédant un groupe fonctionnel déterminé. De tels capteurs permettent de détecter et d'identifier la présence d'un COV appartenant à une famille déterminée mais ne permettent pas de différencier des COV appartenant à une même famille.

Dans un mode de réalisation particulier, la matrice de capteurs comprend des capteurs spécifiques à chaque famille de COV. Dans ce cas, la réponse de la matrice de capteurs permet de conclure sur la présence ou l'absence d'un COV dans une portion d'éluât donnée, mais ne suffit pas à elle seule à déterminer la nature du COV détecté. En revanche, la réponse de la matrice de capteurs permet de déterminer à quelle(s) famille(s) appartient le COV détecté et la connaissance du temps de rétention de la portion d'éluât considérée permet de savoir quel COV cible peut être présent dans ladite portion d'éluât. Ainsi, il est possible de déduire la présence ou l'absence des COV cibles en combinant les informations fournies par le temps de rétention et la matrice de capteurs.

Dans un autre mode de réalisation, la matrice comprend un ensemble de capteurs permettant l'obtention d'une empreinte globale spécifique à chaque COV cible. Par empreinte globale, on entend la combinaison des réponses de l'ensemble des capteurs de la matrice. Dans ce cas, bien que chaque capteur de la matrice ne soit pas spécifique à un seul COV cible, la réponse combinée de plusieurs capteurs permet d'identifier de manière spécifique chaque COV cible. Ainsi, il est possible de déduire la présence ou l'absence des COV cibles des informations fournies par la matrice de capteurs.

Dans un autre mode de réalisation, la matrice de capteurs comprend des capteurs spécifiques à chaque COV cible. Dans ce cas, la matrice de capteurs comprend autant de capteurs que de COV cibles et la réponse de chaque capteur spécifique permet de conclure individuellement sur la présence ou l'absence du COV cible dont il est spécifique.

Avantageusement, le module de détection comprend également une chambre de confinement enfermant la matrice de capteurs. Cette chambre permet le confinement des couches sensibles des capteurs afin de les exposer uniquement aux échantillons à analyser. Avantageusement, la chambre de confinement est réalisée dans un matériau non émissif ou peu émissif de COV dans les conditions d'analyse, tel que le l'acier inoxydable ou le polytétrafluoroéthylène (PTFE), afin d'éviter la contamination de l'échantillon à analyser.

Dans un mode de réalisation particulier, le dispositif de l'invention comprend en outre un module de traitement de l'information telle que la carte de commande. Celui-ci est capable d'interpréter les signaux émis par chaque capteur et de déduire la présence ou l'absence de chaque COV cible. De préférence, le module de traitement de l'information détermine la présence ou non d'une contamination fongique. Cette détermination peut se faire par exemple par le calcul d'un indice de contamination fongique tel que défini dans la demande de brevet FR 2913501.

Les méthodes classiques de détection et/ou d'identification mettent en oeuvre des appareillages complexes tels que des spectromètres de masse, des spectromètres infrarouge, des détecteurs à ionisation de flamme ou des détecteurs à conductivité thermique qui sont difficiles à miniaturiser. Ce dispositif a l'avantage de pouvoir être miniaturisé et de pouvoir être utilisé sans l'intervention d'un technicien spécialisé.

Le dispositif de l'invention présente donc un avantage quant à sa taille et permet de réduire considérablement l'intervalle de temps entre les mesures successives et/ou le temps de réponse de la mesure. La durée d'une mesure avec le dispositif de l'invention est typiquement de 10 à 180 min, de préférence 30 à 120 min. Un tel dispositif offre donc la possibilité de mettre en place une stratégie efficace de surveillance des contaminations fongiques avec un intervalle de temps faible entre les mesures. Ainsi une procédure d'alerte peut être envisagée afin de rechercher et traiter les contaminations à leurs premiers stades de développement. De plus, les systèmes de contrôle de l'air ambiant, tels que les VMC, peuvent être asservis au dispositif de l'invention pour empêcher ou limiter le développement fongique.

En particulier, la présente invention concerne également un procédé de détection d'une contamination fongique dans un environnement intérieur mis en oeuvre par le dispositif de l'invention et comprenant:

- le prélèvement d'un échantillon de COV dans l'environnement intérieur ;

- la séparation des COV prélevés ; et

- la détection des COV fongiques présents.

Le procédé de l'invention comprend le prélèvement d'un échantillon de molécules cibles, de préférence de COV dans l'environnement intérieur. Pour ce faire, le dispositif de l'invention est disposé dans l'environnement intérieur et le prélèvement est effectué par contact entre le module de préconcentration et l'air ambiant.. le prélèvement est effectué par convection forcée provoquant le passage de l'air ambiant au travers du module de préconcentration. Le débit de l'air ambiant passant au travers du module de prélèvement est par exemple de 10 à 1000 mL/min. Le prélèvement de l'échantillon dure alors entre 5 et 60 min. Le prélèvement est de préférence effectué par adsorption des COV sur un matériau adsorbant. Dans ce cas, le procédé de l'invention comprend également une étape de désorption des COV adsorbés. Celle-ci est effectuée par thermo-désorption dans des conditions bien connues de l'homme du métier.

Le procédé de l'invention comprend également la séparation des molécules cibles, en particulier des COV prélevés. La séparation des COV prélevés est effectuée par le module de séparation. En particulier, les COV prélevés sont séparés par élution sur une micro-colonne chromatographique. Les paramètres optimums de séparation comme la température de la colonne ou le débit de la phase mobile, sont déterminés selon les techniques bien connues de l'homme du métier en fonction de la géométrie de la colonne, de la nature de la phase stationnaire et du gaz vecteur.

Dans un mode de réalisation du procédé selon l'invention, on sélectionne des COV cibles parmi les COV prélevés par le module de séparation. Cette étape est effectuée par le système de sélection au cours de l'élution de l'échantillon sur la micro-colonne chromatographique. Pour ce faire, on procède de la façon suivante. Chaque COV cible élue à une vitesse différente connue pour un système chromatographique donné. On attribue donc un temps de rétention donné à un COV cible. On programme le système de sélection avec ces valeurs. Le système de sélection est alors capable de sélectionner les portions d'éluât ayant un temps de rétention correspondant aux COV cibles. Ces portions d'éluât sont alors envoyées sélectivement vers le module de détection. Les portions d'éluât ne correspondant pas aux valeurs préprogrammées sont éliminées. Par conséquent, seules sont détectées par le module de détection la présence ou l'absence des COV cibles.

Le reste de l'éluât étant évacué du circuit d'analyse, cela permet d'éviter les phénomènes d'hystérésis et/ou de saturation des capteurs du module de détection que pourrait provoquer la présence des COV non cibles qui ont généralement une concentration bien supérieure à celle des COV d'origine fongique.

Les COV cibles sont de préférence choisis dans le groupe consistant en le 1-octèn-3-ol, le 1,3-octadiène, le méthyl-2-éthylhexanoate, le 2-méthylfurane, le 3-méthylfurane, le 3-méthyl-1-butanol, le 2- méthyl-1-butanol, le $\alpha$-pinène le 2-heptène, le diméthylsulfure, le 4-heptanone, le 2(5H)-furanone, le 3-heptanol, le méthoxybenzène et le 2-éthylhexanol, et leurs mélanges.

Avantageusement, le procédé de l'invention comprend également la détermination d'un indice de contamination fongique, par exemple en utilisant le procédé comme défini dans la demande de brevet FR 2913501.

Le procédé selon l'invention est de préférence utilisé en continu. Avantageusement, la durée d'un cycle de mesure est de 10 à 180 min, de préférence 30 à 120 min.

## BREVE DESCRIPTION DES FIGURES

D'autres caractéristiques, détails et avantages de l'invention ressortiront à la lecture de la description qui suit, en référence aux figures annexées, qui illustrent :

- Figure 1: un schéma du dispositif de détection selon une variante préférée de l'invention.
- Figure 2 : une représentation des motifs d'alignements de la microstructure de prétraitement ;
- Figure 3 : une représentation des 3 niveaux de masque pour la réalisation d'une micro-colonne chromatographique ;
- Figure 4 : une représentation des 3 niveaux de masque pour la réalisation d'une microstructure de préconcentration ;
- Figure 5 : une représentation schématique du procédé de fabrication des micromodules prétraitement ;
- Figure 6 : une représentation schématique du procédé de fabrication des électrodes interdigitées du module de détection ;
- Figure 7 : une représentation du masque pour la réalisation des puces contenant les électrodes interdigitées du module de détection ;
- FIG. 8: un chromatogramme des émissions de la chambre contenant les 8 traceurs (Chaîne Hewlett Packard - mode SIM) pour la validation du module de concentration,
- FIG. 9 : un chromatogramme obtenu à partir de l'injection de 5 $\mu$L de la solution mère contenant 8 traceurs dans de l'éthanol (HP)) pour la validation du module de séparation,
- Figure 10A : un montage de « pont de Wheatstone » et un montage d'amplification pour le traitement des informations des capteurs du module de détection ;
- Figure 10B : un montage d'amplification pour le traitement des informations des capteurs du module de détection ;
- FIG 10C : un schéma de la réponse du film Ppy/octane sulfonate (0,3M) aux 8 COVm, dans de l'éthanol et de l'eau ;
- FIG. 10D : un schéma de la réponse des films Ppy / octane sulfonate (0,3M) et PEDOT-PSS ; et
- FIG. 10E: un schéma de la réponse du film PEDOT-PSS.
- FIG. 10F: un schéma de la réponse du film PEDOT-PSS pour des alcools encombrés.
- FIG. 11 : un schéma de principe du système d'analyse comprenant le dispositif de détection et l'interface de commande ;
- FIG. 12 : une représentation schématique des états des éléments du système d'analyse ;
- FIG. 13 : un schéma de l'interface de commande du dispositif de détection et
- FIG. 14 : un organigramme de fonctionnement du système d'analyse.

Pour plus de clarté, les éléments identiques ou similaires sont repérés par des signes de référence identiques sur l'ensemble des figures.

## DESCRIPTION DETAILLEE DE MODES DE REALISATION

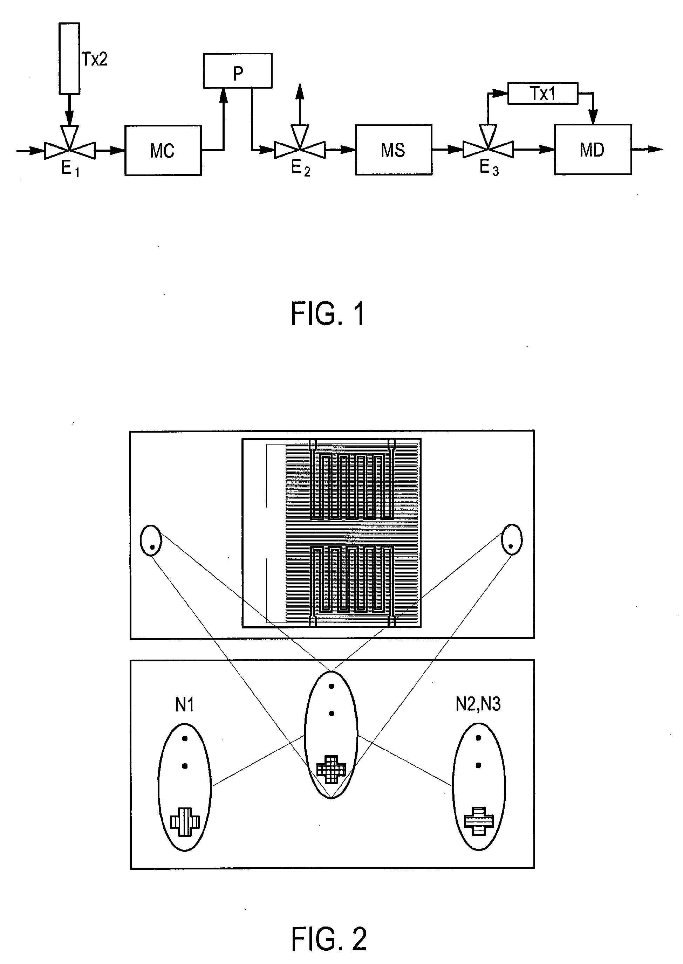

En référence à la figure 1, le dispositif selon une variante préférée de l'invention comprend un module de concentration MC, un module de séparation MS et un module de détection MD, les modules étant successivement en aval les un des autres. Le dispositif comprend en outre une électrovanne E1 qui présente deux positions a et b en amont du module de concentration. Cette électrovanne E1 permet une entrée d'un prélèvement d'air lorsqu'elle est en position a, et une entrée d'un air filtré par un tube tenax Tx2 lorsqu'elle est en position b. Le dispositif comprend également une électrovanne E2 entre le module de concentration et le module de séparation. Cette électrovanne E2 présente deux positions a et b et permet une orientation d'un flux d'air provenant du module de concentration MC vers le module de séparation MS lorsqu'elle est en position b, et une sortie de ce flux vers l'extérieur du dispositif lorsqu'elle est en position a. Les flux d'air sont générés par une pompe P située ici entre le module de concentration et l'électrovanne E2. Le dispositif comprend également une électrovanne E3 entre le module de séparation MS et le module de détection MD. Cette électrovanne E3 présente deux positions a et b et permet une orientation d'un flux d'air provenant du module de séparation MS directement vers le module de détection MD lorsqu'elle est en position a, et une orientation de ce flux vers le module de détection MD par l'intermédiaire d'un tube tenax Tx1 lorsqu'elle est en position b.

Les exemples de réalisation suivants illustrent la présente invention, sans en limiter en aucune façon la portée.

EXEMPLE 1 : PREMIER MODE DE REALISATION DU DISPOSITIF

Le module de préconcentration comprend un micro-préconcentrateur gravé sur une plaque de silicium par un procédé DRIE. Le micro-préconcentrateur est composée de 20 sillons de 6 cm de long, de section rectangulaire de 500 $\mu$m de largeur et 250 $\mu$m de longueur, et présente un volume utile de 0,15 cm$^3$. Les sillons sont remplis de particules de résine à base d'oxyde de 2,6-diphényle commercialisé sous le nom TENAX® TA ayant un diamètre moyen de 120 $\mu$m, une surface spécifique de 35 m$^2$/g, une porosité de 2,4 cm$^3$/g et une taille de pores moyenne de 200 nm. Le micro-préconcentrateur est refermé par une plaque de verre collée sur la surface comportant les sillons de la première plaque.

Une micro-colonne chromatographique a été gravée sur une plaque de silicium par un procédé DRIE. La micro-colonne est composée d'un sillon de 5 m de long, de section rectangulaire de 150 $\mu$m de largeur et 200 $\mu$m de longueur. Le sillon est agencé sous forme de lacets parallèles (ou serpentin) présentant des coudes sous forme d'arc de cercle afin d'éviter la formation d'angles morts. Une phase stationnaire de PDMS, diméthylpolysiloxane (Sylgard® 184, commercialisé par la société Dow corning), est présente à l'intérieur de la micro-colonne. La micro-colonne est refermée avec une seconde plaque de verre collée sur la surface comportant le sillon de la première plaque.

Le module de détection comprend une matrice de capteurs composée de 4 capteurs polymères. Les capteurs polymères ayant une affinité avec les COV d'origine fongique (respectivement le PEDOT-PSS, le polypyrrole/octane sulfonate de sodium, le polypyrrole/perchlorate de lithium et le polybifluorène) déposé sur des paires d'électrodes interdigitées. La matrice de capteurs est disposée dans une chambre de confinement en acier inoxydable et un joint PTFE.

Les différents éléments sont reliés entre eux et au système de circulation par des connecteurs NanoPort™.

EXEMPLE 2 : ETALONNAGE DE LA MICRO-COLONNE DU PREMIER MODE DE REALISATION

Pour l'étalonnage, la matrice de capteurs du dispositif de l'exemple 1 a été remplacée par un spectromètre de masse.

Les paramètres expérimentaux de la chaîne d'analyse sont rassemblés dans le tableau 1.

Tableau 1 : Caractéristiques du GC/MS

| Paramètres | Conditions analytiques |
|---|---|
| Thermo-désorbeur | Turbomatrix ATD (Perkin Elmer) |
| Température de désorption | 370 °C |
| Débit de désorption | 50 mL/min Azote N50 |
| Durée de désorption | 15 min |
| Température du piège froid (Tenax TA) | -30 °C |
| Température d'injection (40°C/s) | 300 °C |
| Température de la ligne de transfert | 220 °C |
| Chromatographe gaz / Spectromètre de masse | Autosystem XL / Turbomass (Perkin Elmer) |
| Micro-colonne | Sylgard 184 |
| Gaz vecteur | Hélium N60 |
| Pression constante | 37,5 psi |
| Cycle de température | 40 °C pendant 2 min<br>1 °C/min. jusqu'à 41 °C<br>Plateau de 2 min<br>0,3°C/min. jusqu'à 44°C pendant 2 min<br>1°C/min. jusqu'à 47°C<br>Plateau de 2 min |
| Paramètres du spectromètre de masse | Quadripôle mode EI, balayage (33-400) |

Des échantillons des COV cibles ont été passés dans la micro-colonne afin de déterminer les temps de rétention de chaque COV cible.

Les temps de rétention de chaque COV cible sont répertoriés dans le tableau 2.

Tableau 2

| Composes | Temps de rétention (min) |
|---|---|
| 1-octen-3-ol | 4.7 |

(suite)

| Composes | Temps de rétention (min) |
|---|---|
| 1,3-octadiène | 1.6 |
| méthyl-2-éthylhexanoate | 9.1 |
| 2-éthylhexanol | 5.7 |
| $\alpha$-pinène | 2.7 |
| 2-méthylfurane | 0.5 |
| 3-méthylfurane | 0.5 |
| 3-méthyl-1-butanol | 1.4 |
| 2-méthyl-1-butanol | 1.4 |
| 2-héptène | 0.8 |
| 4-heptanone | 2.1 |
| 3-heptanol | 4.8 |
| Méthoxybenzène | 2.6 |

EXEMPLE 3 : METHODES EXPERIMENTALES

3.1 DISPOSITIF EXPERIMENTAL POUR CAPTEURS POLYMERES

3.1.A Système d'acquisition de données

Les expérimentations ont été réalisées à partir d'un système permettant l'acquisition de signaux issus d'une carte composée de polymères conducteurs constituant le coeur du système.

Les chambres d'émissions sont placées en dehors du système. Un système de filtration composé d'un tube TENAX -Tx- est situé en amont de la chambre d'émission et assure un renouvellement d'air « propre » (fuite). En aval, un tube en PTFE permet la connexion entre la chambre d'émission et une électrovanne 3 voies. L'ensemble des raccords est également en PTFE. L'électrovanne 3 voies (commercialisé par la société BIO-CHEM-VALVE CORP) permet de sélectionner une voie de référence (air filtré sur charbon actif), une voie d'échantillonnage (chambre d'émission) ou une voie de nettoyage (mélange 1-butanol / eau).

Une pompe (commercialisé par la société ESCAP) permet de transférer l'air de différentes ambiances à 147 $\pm$ 1 mL.min$^{-1}$ vers la matrice de capteurs confinée dans une chambre PTFE (20x25x5 mm de dimensions intérieures soit un volume total de 2,5 mL).

Une carte adaptée à ce système a ainsi été réalisée spécifiquement pour cette étude. Cette carte est constituée de 12 paires d'électrodes en or (avec une couche d'accroche en chrome) déposées sur un wafer en verre (on parle encore de plaque ou de microplaque).

Des polymères sont ensuite déposés entre chaque paire d'électrodes par électropolymérisation ou par drop-coating (dépôt d'une goutte pour les polymères en solution).

Le système est commandé aux cours du temps. Il permet d'observer l'évolution de la résistivité des capteurs ($R_{sample}$), ainsi que de récupérer informatiquement ces données pour les traiter. De l'air filtré sur charbon actif, est placé en référence afin de déterminer la ligne de base ($R_{référence}$). Un mélange 1-butanol / eau (1/50 ; v/v) permet le nettoyage complet des capteurs suite à une exposition à une chambre d'émission. En effet, ce mélange permet de saturer le capteur tout en permettant un retour rapide à la ligne de base.

Les résultats sont présentés sous forme de différences fractionnelles de résistances :

$$\% \frac{dR}{R} = \frac{\left(R_{sample} - R_{référence}\right)}{R_{référence}} \times 100$$

3.1.B Dépôt de couche polymères

Les polymères conducteurs peuvent être synthétisés à partir d'une large gamme de monomères dans divers solvants en présence d'un grand nombre de contre-ions. Les expérimentations ont donc consisté à déposer différents polymères conducteurs, dopés avec différents contre-ions ainsi que des mélanges de ces polymères, sur les électrodes de la matrice du système.

Les polymères en solution ont été déposés à l'aide d'un cône de micropipette. Les polymères disponibles sous forme de poudre ont été solubilisés dans du chloroforme puis déposés également à l'aide d'un cône de micropipette. Ces polymères en solution ont ensuite été dopés avec des vapeurs de diiode (12) pendant 2 heures. Le PEDOT-PSS est un polymère en solution aqueuse déjà conducteur et n'a donc pas besoin d'être dopé avec les vapeurs de diiode.

Le dépôt des polymères insolubles se fait par électropolymérisation jusqu'à percolation (jonction de polymère entre les deux électrodes) à l'aide d'un montage à 3 électrodes et de la solution de monomère (concentration de 0.05 mol.L$^{-1}$) avec un électrolyte (concentration de 0.1 mol.L$^{-1}$).

EXEMPLE 4 : CARACTERISATION DES COUCHES SENSIBLES

4.1 DIFFERENCIATION D'AMBIANCES PAR DES CAPTEURS POLYMERES

4.1.A Caractérisation à partir de souches fongiques

Des essais préliminaires, réalisés à partir de différentes souches de l'étude, ont permis d'obtenir des réponses spécifiques de capteurs polymères soumis à des environnements moisis. L'ensemble des profils obtenus avec les différentes souches (répété 3 fois) montre un comportement différent de la réponse des capteurs entre un environnement contaminé et un environnement stérilisé. Suite à cette observation, une caractérisation plus précise des réponses de capteurs polymères a été réalisée avec deux espèces de moisissures fréquemment rencontrées dans tous types d'environnements intérieurs : *Penicillium brevicompactum* et *Aspergillus niger.*

Le protocole expérimental utilisé pour la caractérisation des réponses consiste à faire passer différents échantillons d'air sur les 12 capteurs polymères présents sur la carte du système pendant 20 min. Parmi les 14 polymères et mélanges de polymères testés (tableau 3), 5 montrent un comportement différent du signal en fonction des types d'échantillons (air filtré, chambre témoin, chambre contaminée). Des essais ont été réalisés à partir de polymères conducteurs suivants. La liste des polymères ainsi que les mélanges (superposition de différentes couches) utilisés pour la distinction d'environnements non-contaminés et contaminés est présentée dans le tableau 3.

## Tableau 3 : Liste des polymères conducteurs testés.

| Polymère en solution | |
|---|---|
| Poly(éthylènedioxythiophène)-poly(styrènesulfonate) (PEDOT-PSS) | |
| **Polymères en solution (dopés par I$_2$)** | |
| Polymères seuls | Mélanges de polymères |
| Poly (biFluorène-EDOT-Carbazole) | PEDOT-PSS + Poly (biFl-EDOT-Cz) |
| Poly (4-fluorophényl) thiophène | PEDOT-PSS + Poly (4-fluorophényl) thiophène |
| Copolymère (biFluorène-EDOT-Carbazole) (COPO (biFl-EDOT-Cz)) | PEDOT-PSS + COPO (biFl-EDOT-Cz) |
| Poly (EDOT-di-Cz) | PEDOT-PSS + Poly (EDOT-di-Cz) |
| Poly(3-hexylthiophène) (P3HT) | PEDOT-PSS + P3HT |
| **Polymères électrodéposés** | |
| Polymères seuls | |
| Polypyrrole + perchlorate de lithium | |
| Polypyrrole + para-toluène sulfonate de sodium | |
| PEDOT + perchlorate de lithium | |

Les capteurs ayant donné des résultats exploitables sont à base de PEDOT-PSS, de Polypyrrole + perchlorate de lithium, de PEDOT-PSS et de COPO, d'unité bifluorène dibromé.avantageusement dopé par des vapeurs de diiode et de Ppy/octane sulfonate (0,3M) dans de léthanol et de l'eau.

Les autres polymères n'ont montré soit aucune réponse soit des réponses variables non spécifiques des stimuli appliqués.

Un protocole de nettoyage permettant une désorption complète des COV adsorbés, est préférentiellement réalisé par la suite afin d'éviter cette dérive dans le cas d'une utilisation de ces polymères dans un système de capteurs dédié à la détection des moisissures pour des applications in situ.

Durant les différents tests, un phénomène de dilution de l'air contaminé des chambres a également été constaté du fait des prélèvements successifs. Cela permet d'expliquer les variations entre deux prélèvements dans une même chambre.

Les COV émis par les moisissures sont des molécules polaires (alcool, cétones, composés sulfurés). L'hypothèse émise sur le mécanisme d'interaction serait donc une interaction de ces fonctions polaires avec les atomes d'oxygène (O), de soufre (S) et d'azote (N) présents sur les polymères.

EXEMPLE 5 : DEUXIEME MODE DE REALISATION.

Dans une problématique d'analyse de composés dans l'air, les limites principales des systèmes multi-capteurs sont leurs fortes sensibilités à l'humidité, la dérive et la pollution des couches sensibles des capteurs. Cependant, l'utilisation d'une matrice de capteurs permet un échantillonnage rapide, simple, non envahissant et non destructif, pour la détection et l'identification de composés volatils, sans qu'une formation complexe pour l'utilisateur ne soit nécessaire.

6.1 MATERIEL BIOLOGIQUE ET SUPPORT DE CROISSANCE

L'espèce fongique sélectionnée pour ces essais est une souche de l'Institut d'Hygiène et d'Epidémiologie de Bruxelles (IHEM) : *Aspergillus niger.* Cette souche est conservée à 4°C dans de l'eau ultra pure sur milieu S10 (Bouillon de Sabouraud glucosé à 2 % dilué ; Merck). La culture est alternée sur S10 qui constitue un milieu pauvre (milieu plus proche de la réalité) et sur gélose avoine qui est un milieu riche en nutriments. La culture finale est obtenue après 7 jours à 25°C sur gélose avoine.

Quel que soit le milieu nutritif, les cultures sont incubées à 25°C dans l'obscurité. Cette espèce a été choisie car elle est fréquemment retrouvée dans les environnements intérieurs et émet, lors de son développement, tous les traceurs de l'indice de contamination fongique.

Le support de croissance employé est de la toile de verre à peindre. Ce matériau est découpé et stérilisé (121°C, 45 min, chaleur humide) avant d'y ajouter de l'eau distillée.

6.2 CHAMBRES D'EMISSIONS DE COV SPECIFIQUES

Les essais sur les modules de concentration et de séparation ont également été réalisés à partir de chambres d'émission de 300 mL identiques à celles utilisées précédemment dans ce mode de réalisation pour le développement des indices de contamination. Des standards de cibles chimiques ont alors été déposés dans ces chambres afin de tester les différents micromodules. Pour cela, huit composés identifiés comme traceurs d'un développement fongique ont été utilisés. La liste de ces standards (commercialisés par la société SIGMA-ALDRICH) est décrite au tableau 4.

## Tableau 4 : Liste des standards pour la validation des modules de prétraitement

| Composé | Pureté (%) |
|---|---|
| 2-méthylfurane | 99 |
| 3-méthyl-1-butanol | 99 |
| 2-méthyl-1-butanol | 99 |
| 4-heptanone | 98 |
| 3-heptanol | 99 |
| méthoxybenzène | 99 |
| α-pinène | 98 |
| 1-octèn-3-ol | 98 |

Ces 8 traceurs ont été mis en solution dans de l'éthanol afin d'obtenir une concentration à 5 g.L$^{-1}$.

Le support de croissance employé est de la toile de verre. Ce matériau est découpé et stérilisé (121°C, 45 min, chaleur humide) avant d'y ajouter de l'eau distillée. Chacune des chambres contient, 50 mL de billes de verre et 5 mL d'eau distillée. Après disposition du support dans les chambres, le taux de charge obtenu est de 7.10-2 cm$^2$/cm$^3$.

A partir de ces chambres d'émission, trois ambiances ont été développées, une ambiance témoin (absence de contamination), une ambiance contaminée par *Aspergillus niger* et une ambiance contenant une solution des 8 standards. La préparation de la suspension de spores utilisée pour la contamination par *Aspergillus niger* est réalisée en versant 50 mL d'eau ultra pure sur la souche repiquée sur avoine.

Pour le conditionnement des différentes chambres, de l'air filtré sur charbon actif (élimination de COV présents avant le début de la croissance) est passé pendant 30 min. Les chambres sont ensuite placées dans une étuve durant 7 jours à 25°C et à l'obscurité.

6.3 ECHANTILLONNAGE ET ANALYSES DES COV

6.3.A Echantillonnage des COV

Pour l'échantillonnage et l'analyse des COV lors de la phase de validation des microstructures, deux chaînes analytiques ont été utilisées. Ainsi, deux techniques de prélèvement sont préférées afin de permettre un échantillonnage compatible avec le système d'injection de chacune des deux chaînes analytiques.

Concernant la première chaîne analytique -GC/MS 1- (commercialisée par la société Perkin Elmer), l'échantillonnage est réalisé à partir de tube Tenax. Comme pour la partie de développement des indices de contamination, les échantillonnages sur tube Tenax -Tx- sont effectués en dynamique avec un débit de 100 mL.min$^{-1}$ durant 30 min dans une chambre à l'aide d'une pompe à air FL-1001, commercialisée par la société Flec.

Ainsi, les COV sont piégés dans des tubes en acier inoxydable, contenant un adsorbant solide approprié pour les COV contenant 4 à 18 atomes de carbone, le Tenax TA (commercialisé par la société Supelco).

Cet adsorbant est un polymère poreux fondé sur de l'oxyde de 2,6-diphénylène dont la granulométrie varie de 0,18 mm à 0,25 mm (60 mailles à 80 mailles). Un nettoyage préalable du Tenax TA par conditionnement thermique, sous un flux d'azote est avantageusement réalisé.

Le tube Tenax est ensuite désorbé avec un thermo-désorbeur automatique permettant ainsi de libérer les COV piégés. L'échantillon ainsi désorbé est injecté directement dans la colonne. La désorption thermique est une technique d'extraction de composés organiques volatils à partir d'une matrice solide, par chauffage d'un échantillon balayé par un flux de gaz inerte. Les composés sont adsorbés sur un piège froid à -30°C puis désorbés à 300°C avant d'être dirigés vers la colonne chromatographique où ils seront séparés.

La seconde chaîne -GC/MS 2- (commercialisé par la société Hewlett Packard) ne comprend pas de thermo-désorbeur. Dans ce cas, l'échantillonnage de COV est réalisé à partir de vials (flacon en verre d'un volume de 2 ml), dans lesquels sont recueillis les échantillons de gaz à analyser. Les émissions issues des chambres sont alors prélevées par une pompe à membrane SP 725 EC avec un débit de 6,2 mL.min$^{-1}$ durant 30 min dans une chambre contenant un vial de prélèvement.

L'échantillon de gaz est alors confiné dans le vial permettant ensuite à l'injecteur automatique de la chaîne d'analyse de prélever un volume d'air à l'aide d'une seringue dans un des vials présents sur le passeur automatique.

6.3.B Description des chaînes analytiques

Les 2 chaînes analytiques, GC/MS 1 et 2, utilisées pour analyser les COV consistent en la combinaison de deux techniques :

- La chromatographie en phase gazeuse (GC) utilisée pour séparer les COV.

- La spectrométrie de masse (MS) employée pour identifier ces composés.

Les caractéristiques des deux chaînes d'analyse, Perkin Elmer et Hewlett Packard, utilisées pour la validation des microstructures sont précisées respectivement dans les tableaux 5 et 6.

## Tableau 5 : Caractéristiques du GC/MS 1 (Perkin Elmer).

| Paramètres | Conditions analytiques |
|---|---|
| Thermo-désorbeur | Turbomatrix ATD (Perkin Elmer) |
| Température de désorption | 370 °C |
| Débit de désorption | 50 ml/min Azote N50 |
| Durée de désorption | 15 min |
| Température du piège froid (Tenax TA) | -30 °C |
| Température d'injection (40°C/s) | 300 °C |
| Température de la ligne de transfert | 220 °C |
| Chromatographe gaz / Spectromètre de masse | Autosystem XL / Turbomass (Perkin Elmer) |
| Colonne capillaire | CP-SIL PONA CB |
| caractéristiques géométriques de la colonne : longueur; diamètre interne; épaisseur de la phase stationnaire | 50 m ; 0,21 mm ; 0,5 µm |
| Gaz vecteur | Hélium N60 |
| Pression constante | 37,5 psi |
| Cycle de température | 40 °C pendant 2 min. 1°C/min. jusqu'à 41°C plateau de 2 min. 0,3 °C/min. jusqu'à 44°C pendant 2 min. 1°C/min. jusqu'à 47°C plateau de 2 min. |
| Paramètres du spectromètre de masse | Quadripôle mode EI, balayage (33-400) |

## Tableau 6 : Caractéristiques du GC/MS 2 (Hewlett Packard).

| Paramètres | Conditions analytiques |
|---|---|
| Température d'injection (40°C/s) | 300 °C |
| Température de la ligne de transfert | 200 °C |
| Chromatographe gaz / Spectromètre de masse | HP 6890 / HP 5973 |
| Colonne capillaire | HP-SMSUI (phase : 5% Phényl 95% Méthylpolysiloxane) |
| caractéristiques géométriques de la colonne : longueur; diamètre interne; épaisseur de la phase stationnaire | 30 m; 0,25 mm; 0,25 µm |
| Gaz vecteur / débit | Hélium / 0.5 mL/min |
| Cycle de température | Isotherme 40 °C durant 10 min |
| Interface | 200 °C |
| Paramètres du spectromètre de masse | Quadripôle mode EI, balayage (20-700) |
| Température MS source | 230°C |
| Température MS quadripôle | 150°C |

Pour les 2 chaînes analytiques, les spectres obtenus sont comparés à une bibliothèque de spectres de masse (NIST, 1998).

Deux modes d'analyse ont été utilisés pour la détection des composés :

- Le mode dit "de balayage" ou "fullscan", employé lorsque l'on souhaite enregistrer des spectres dit "de source", c'est-à-dire des spectres où sont présents tous les ions produits dans la source à un instant donné.

- Le mode "SIM" ("Single Ion Monitoring"), qui consiste à ne détecter qu'un (ou quelques) ion(s). Le spectromètre de masse fonctionne donc comme un filtre. Celui-ci est programmé pour ne détecter que quelques ions caractéristiques des analytes étudiés (1 à 4, en général). L'augmentation du signal associé à la détection des analytes permet un gain en sensibilité tout en diminuant le bruit de fond chromatographique. Avec un quadripôle, la durée du balayage (dwell) des ions est proportionnelle à la gamme de rapports m/z balayée. Opérer sur peu de valeurs de m/z augmente donc considérablement le temps imparti à la détection des ions correspondants, comparativement au mode "fullscan". Le mode "SIM" est utilisé lorsque de faibles concentrations d'échantillon sont injectées.

Ainsi, lors d'un prélèvement sur tube Tenax, un split en sortie de la colonne permet d'injecter 3 % du volume de l'échantillon prélevé soit 90 mL afin de protéger le détecteur en cas de concentration trop importante d'un composé.

Dans le cas d'un prélèvement avec vial, le volume injecté est de 5 μL. Il y a donc un facteur de dilution de 18 000 en comparaison avec une analyse d'un échantillon prélevé sur tube Tenax. Le tableau 7 répertorie les caractéristiques de prélèvement en fonction des deux modes d'échantillonnage utilisés.

## Tableau 7 : Caractéristiques des prélèvements.

| Mode de prélèvement | Tube Tenax | Vial |
|---|---|---|
| Débit (mL.min$^{-1}$) | 100 | 6.2 |
| Durée (min) | 30 | 30 |
| V$_{prélèvement}$ (mL) | 3000 | 186 |
| V$_{injection}$ | 90 mL | 5 μL |

### 6.4 DISPOSITIFS DE VALIDATION DES MODULES

#### 6.4.A Module de préconcentration

La microstructure de concentration développée dans ce mode de réalisation se compose d'un substrat de silicium dans lequel des sillons de 60 mm de longueur pour une largeur de 500 μm sont gravés. La fusion avec un substrat en verre permet la réalisation de cavités closes. La microstructure est ensuite fonctionnalisée avec des grains de Tenax avec un diamètre moyen de 120 μm. Des connecteurs fluidiques adaptés à ce type de microstructures permettent d'équiper les orifices d'accès des micromodules de capillaires afin de permettre la connexion avec une pompe et permettre une circulation d'air à travers la structure.

Le protocole de test concernant la microstructure de concentration implique préférentiellement l'utilisation de deux types d'échantillons, un échantillon avec les traceurs (chambre d'émission contenant le mélange de 8 traceurs) et un échantillon avec une contamination fongique (chambre d'émission contenant de la toile de verre contaminée par *Aspergillus niger*).

Le protocole de test consiste a prélevé de l'air des différentes chambres à travers le préconcentrateur pendant 30 min à un débit de 6,2 mL.min$^{-1}$ par l'intermédiaire d'une pompe à membrane SP 725 EC. L'échantillon issu du préconcentrateur est ensuite désorbé à 140°C pendant 30 min et extrait via la pompe dans une chambre contenant un vial de prélèvement. L'échantillon issu du préconcentrateur contenu dans le vial est alors analysé sur une chaîne d'analyse GC/MS.

#### 6.4.B Module de séparation

La microstructure de séparation développée dans ce mode de réalisation se compose également d'un substrat de silicium dans lequel un sillon d'une longueur de 5 m de longueur, pour une largeur de 150 μm et une profondeur de 200 μm, est gravé. La fusion avec un substrat en verre permet la réalisation du canal. La microstructure est ensuite fonctionnalisée avec une phase stationnaire composée de PDMS pour permettre la rétention des molécules la traversant. Comme pour la microstructure de concentration, des connecteurs fluidiques adaptés permettent d'équiper les orifices d'accès des micromodules de capillaires afin de permettre la connexion avec une pompe et permettre la circulation du flux d'air à travers la structure.

Une fois la micro-colonne chromatographique réalisée et fonctionnalisée, des essais ont été réalisés afin de vérifier son efficacité de rétention et de séparation de différents composés cibles de l'étude.

Pour cela, le banc d'analyse de chromatographie en phase gazeuse utilisé lors du développement des indices de contamination, a été utilisé en remplaçant la colonne chromatographique classique par la micro-colonne. Le spectromètre de masse a été employé en sortie pour identifier les composés.

Deux types d'échantillons ont également été utilisés dans le protocole de test de la micro-colonne, un échantillon avec les traceurs (chambre d'émission contenant le mélange de 8 traceurs) et un échantillon avec une contamination fongique (chambre d'émission contenant de la toile de verre contaminée par *Aspergillus niger).* Les échantillons d'air des différentes chambres sont prélevés à l'aide de tube Tenax à un débit de 100 mL.min$^{-1}$ pendant 30 min par l'intermédiaire d'une pompe (Air Pump 1001, Flec). Les tubes sont ensuite placés dans le thermo-désorbeur automatique de la chaîne d'analyse GC/MS pour permettre leurs analyses. Les échantillons sont ainsi séparer par la micro-colonne et analysés en sortie par spectrométrie de masse afin de vérifier l'efficacité de rétention. Dans le protocole de test de la micro-colonne, la méthode de prélèvement par vial a également été utilisée. Les échantillons ont également été analysé en remplaçant la colonne classique par la micro-colonne dans la seconde chaîne d'analyse GC/MS.

EXEMPLE 6: PROCEDE DE FABRICATION DES MODULES DE PRETRAITEMENT.

7.1.A Eléments constitutifs de micromodules

Afin de définir le procédé de fabrication des micromodules, le premier choix technologique concerne le substrat utilisé pour la gravure des motifs. Le silicium constitue un matériau de base très largement utilisé pour la réalisation de microsystèmes de part ses caractéristiques mécaniques et électriques. Le silicium monocristallin est abondant et peu cher, et constitue un matériau très adapté à la miniaturisation.

Les dispositifs ont été réalisés sur substrat silicium à partir de plaques (wafers) doubles faces de diamètre 4 pouces (environ 10 cm) et d'épaisseur 500 $\mu$m (entre 475 et 525 $\mu$m).

La réalisation de structures microfluidiques implique de pouvoir réaliser une connexion entre le microsystème et le système de circulation d'air (micropompe, vanne...). La solution retenue dans ce mode de réalisation est l'utilisation de « NanoPort ». Ces connecteurs fluidiques sont adaptés aux microstructures et permettent d'équiper les orifices d'accès des micromodules de capillaires. La mise en place de ces connecteurs nécessite un bon alignement entre l'orifice d'accès de la microstructure et l'orifice du connecteur permettant l'insertion des capillaires. Ces connecteurs se composent, d'un anneau de colle, d'un joint d'étanchéité, du corps du connecteur, et enfin de la « vis » permettant l'insertion du capillaire.

Les tubes utilisés dans ce mode de réalisation pour réaliser les connexions entre les différents modules du système sont des tubes PEEK de diamètre extérieur 1/32 pouces (800 $\mu$m) et de diamètre intérieur 0,008 pouces (200 $\mu$m).

L'utilisation de deux modules que sont une micro-colonne chromatographique ainsi qu'une microstructure de concentration implique de pouvoir en contrôler la température. En effet, la température d'une colonne influence son efficacité et les structures de concentration nécessitent une température de chauffe afin de pouvoir libérer les molécules piégées. Dans cette optique, des résistances de chauffe ont donc été intégrées aux micromodules.

Le matériau le plus souvent utilisé dans la littérature pour ce type d'application est le platine. En plus d'un coefficient de température positif, d'un bon facteur de sensibilité, ce matériau présente une résistivité élevée. Un matériau de forte résistivité présente l'avantage de dissiper de grande quantité de chaleur par effet joule. Le platine est également caractérisé par une grande linéarité en température. L'utilisation du platine nécessite cependant l'utilisation d'une couche d'accroche. Le matériau utilisé pour réaliser cette couche d'accroche est le titane. Les épaisseurs de dépôts sont, respectivement pour le titane et le platine, de 50 et 100 nm.

7.1.B Conception de masques

L'utilisation de la lithographie reste, au travers des technologies silicium, une voie très largement utilisée pour la fabrication de microstructures telles que les micro-colonnes chromatographique ou les microstructures de concentration. Elle permet de réaliser des structures à fortes résolutions. L'approche utilisée généralement pour la réalisation de ce type de structures est dite « top-down », c'est-à-dire qu'à partir du substrat, le procédé vient graver le motif dans la matière.

La lithographie implique de concevoir préalablement des masques, utilisés dans le procédé de fabrication proprement dit, permettant de définir des zones d'expositions et donc les zones de gravure du substrat. La conception de ces masques a été réalisée sous le logiciel Coventor 2008.

Ces masques permettent ainsi de définir la géométrie finale des structures. Pour la réalisation de nos deux modules, « séparation » et « concentration », 3 niveaux de masques ont donc été nécessaires : le premier définissant la géométrie des sillons gravés sur la face avant, le deuxième, sur la face arrière, pour la création des orifices d'accès de la structure, enfin le troisième, pour la réalisation des résistances chauffantes également sur la face arrière.

Masques de la micro-colonne.

Le premier niveau de masque -N1- concerne donc la géométrie des sillons -sill- de la colonne. Plusieurs formes géométriques peuvent être envisagées concernant l'agencement des sillons d'une micro-colonne pour limiter l'encombrement et donc la taille de la structure. Dans ce mode de réalisation, la forme géométrique retenue est une configuration type « serpentin ». Afin de limiter les volumes morts et faciliter le remplissage colonne, les coudes de la colonne entre chaque sillon ont été réalisés sous forme d'arc de cercle permettant la réalisation d'une structure ne présentant pas d'angles droits. Le choix de la longueur de la colonne a été fixé à 5 m. Le choix de cette longueur relativement importante pour ce type de microstructure provient du fait que ce dispositif doit permettre la séparation d'échantillons complexes de différents composés. Le choix d'une longueur importante permet ainsi d'améliorer l'efficacité de la colonne et donc obtenir une meilleure séparation des composés.

Les caractéristiques géométriques de la puce pour la réalisation de la micro-colonne sont reportées dans le tableau 8.

Tableau 8 : Caractéristiques géométriques (en $\mu$m) de la puce micro-colonne

| Sillons | | | | Coudes | | Puce | |
|---|---|---|---|---|---|---|---|
| Longueur | Largeur | Espace entre sillon | Nombre de sillons | Rayons des coudes | Largeur des coudes | Longueur | Largeur |
| 30000 | 150 | 100 | 162 | 125 | 150 | 41400 | 40900 |

Comme pour le choix des résines photosensibles positives ou négatives, dans un procédé de fabrication, le choix de la « polarité » du masque lors de la conception des masques doit être précisé. Ainsi, les données imprimées sur le masque (« digitized data ») doivent être précisées soit en clair (« clear »), avec un fond opaque, soit opaque (« dark »), avec un fond clair. Pour la réalisation de ce premier niveau de masque, la polarité a été choisie avec les données en clair.

Le deuxième niveau de masque -N2- concerne les orifices d'accès à la microstructure. Le choix retenu dans ce mode de réalisation est d'accéder à la structure par la face arrière du substrat via des NanoPorts permettant la connexion entre le microsystème et le système de circulation d'air à l'aide de tube PEEK. Le diamètre interne des tubes PEEK choisi étant de 200 $\mu$m pour un diamètre externe de 800 $\mu$m, les dimensions des orifices d'accès retenues sont de 400 $\mu$m de diamètre afin de faciliter le positionnement du NanoPort. En effet, ces dimensions permettent la mise en butée du tube sur la structure et évitent un risque de chevauchement des orifices du tube et de la structure. La polarité de ce masque a également été choisie avec les données en clair.

Le troisième niveau de masque -N3- concerne la création de résistances chauffantes utilisées pour chauffer et contrôler la température de la structure. L'état de la technique connu de l'homme du métier montre l'importance de la température dans la réalisation de ce type de microstructure de séparation.

La valeur d'une résistance dépend de ces caractéristiques géométriques puisqu'elle est définie par la formule suivante :

$$R = \rho \frac{l}{s} = \frac{\rho}{e} \frac{l}{w}$$

Où p est la résistivité du matériau, I la longueur, w la largeur, S la surface, et e l'épaisseur.

Les résistances chauffantes peuvent être intégrées aux microstructures, néanmoins, dans un premier temps, une enceinte de confinement intégrant une résistance chauffante externe est utilisée.

La présence de motifs d'alignement est préférable lors de phase de lithographie afin de pouvoir superposer les différents masques et d'aligner les différents motifs présents sur chaque masque. Le positionnement des différents masques s'effectue par l'alignement et la superposition de deux figures géométriques. Les motifs d'alignement généralement retrouvés et utilisés dans ce mode de réalisation sont des croix (Figure 2).

Dans ce mode de réalisation, la présence de motifs d'alignement permet notamment de positionner les orifices d'accès -orif- sur la face arrière -post- avec les orifices d'entrée/sortie présents sur la face avant -ant- aux extrémités des microcanaux. Dans ce cas, la précision des motifs d'alignement est d'autant plus importante qu'un décalage de quelques micromètres entre ces deux motifs peut rendre la structure inutilisable. L'utilisation de motifs d'alignement de petites dimensions permet ainsi d'obtenir des précisions d'alignement de l'ordre de 1 μm.

Des motifs d'alignement sont également présents sur le masque des résistances chauffantes RC afin de les positionner sous la surface formée par les microcanaux.

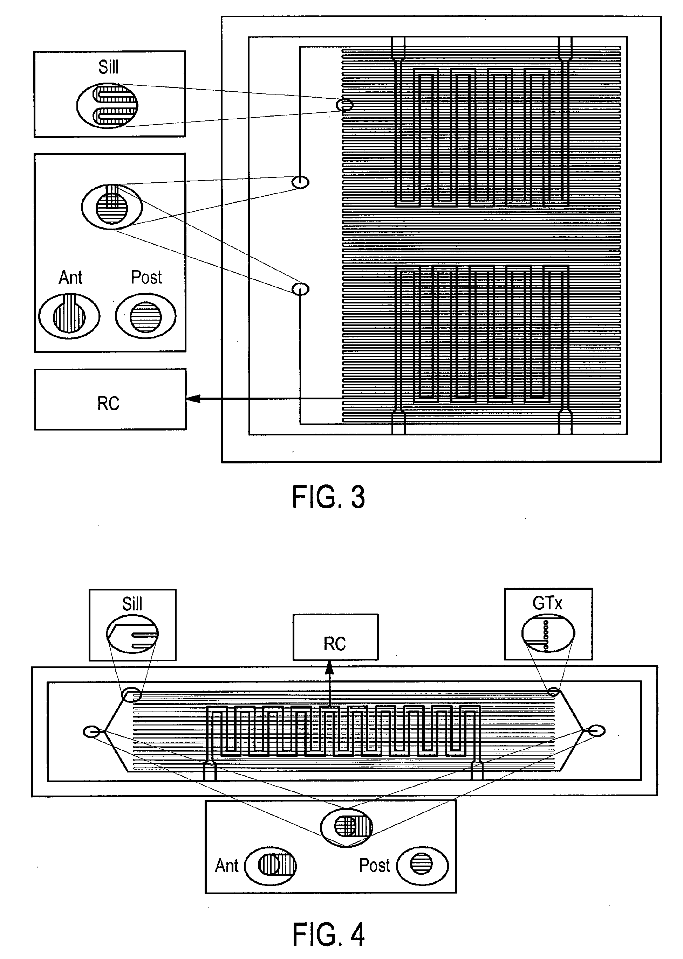

Une image des différents niveaux de masques développés sous Coventor 2008 pour la réalisation d'une puce micro-colonne, est présentée en Figure 3.

Masque du micropréconcentrateur.

Pour la réalisation de microstructure de concentration, plusieurs formes géométriques sont également envisageables. Le matériau adsorbant utilisé dans ce mode de réalisation permettant de piéger les molécules sélectionnées pour la détection fongique, est le TENAX TA. Afin de faciliter le remplissage de la structure avec ce matériau (disponible sous forme de grain), une structure ne contenant aucun coude a été retenue. Les tubes TENAX de prélèvement classiquement utilisés pour la détection des COV dans notre étude contiennent un volume utile de 1 cm$^3$ (longueur de 5 cm pour un diamètre de 0,5 cm), avec des grains de diamètre moyen de 300 μm. Pour la réalisation de la microstructure de préconcentration, la taille des grains utilisée est plus petite (diamètre moyen de 120 μm). L'utilisation d'un diamètre inférieur permet d'augmenter la surface spécifique (6 fois), par conséquent, une réduction du volume utile à 0,25 cm$^3$ (par 4) a été retenue. Des sillons de 60 mm de longueur pour une largeur de 500 μm ont ainsi été développés.

Afin de retenir les grains dans la microstructure lors du passage de l'air, une grille a été fabriquée à l'aide de micropiliers en sortie de la structure. La taille des grains de TENAX insérés dans la structure pouvant atteindre un diamètre minimum de 75 μm, les dimensions de la grille retenue sont des piliers de 56 μm de large pour un espace entres les piliers de 55 μm.

Les caractéristiques géométriques de la puce pour la réalisation du micropréconcentrateur sont reportées dans le tableau 9.

Tableau 9 : Caractéristiques géométriques (en μm) de la puce de préconcentration

| Sillons | | | | Grille Tenax | | Puce | |
|---|---|---|---|---|---|---|---|
| Longueur | Largeur | Espace entre sillon | Nombre de sillons | piliers | Espace entre piliers | Longueur | Largeur |
| 60000 | 500 | 50 | 20 | 56 | 55 | 84600 | 11450 |

Concernant les orifices d'accès sur le deuxième niveau de masque, la solution technologique (NanoPorts) et les caractéristiques géométriques des orifices retenues sont identiques à celles utilisées pour la réalisation de la puce micro-colonne.

Comme pour la conception des masques de la micro-colonne, l'intégration de résistances chauffantes au module de préconcentration est également envisagée et un troisième niveau de masques a donc été développé. Cependant, une enceinte de confinement est également prévue dans un premier temps afin de pouvoir utiliser une résistance chauffante externe.

Une image des différents niveaux de masque développés sous Coventor 2008 pour la réalisation d'une puce de préconcentration, est présentée en Figure 4.

7.1.C Procédé de fabrication des modules de prétraitement

La réalisation de telles structures pose naturellement le problème du choix d'une procédure de fabrication, ou plus exactement d'une technique de gravure, compatible avec de telles résolutions. Les dimensions caractéristiques des canaux sont de l'ordre de la centaine de microns et les sections sont classiquement de forme rectangulaire, semi-circulaire ou circulaire. D'après la bibliographie, la forme de la section pour les colonnes capillaires n'influence pas l'efficacité de séparation de la colonne. Cette remarque permet donc de ne pas s'imposer de contrainte concernant la forme de gravure définie par le procédé de fabrication.

La DRIE est un procédé de fabrication permettant de réaliser des gravures anisotropes profondes à fort rapport d'aspect. Le choix de section rectangulaire réalisée par gravure DRIE a ainsi été retenu.

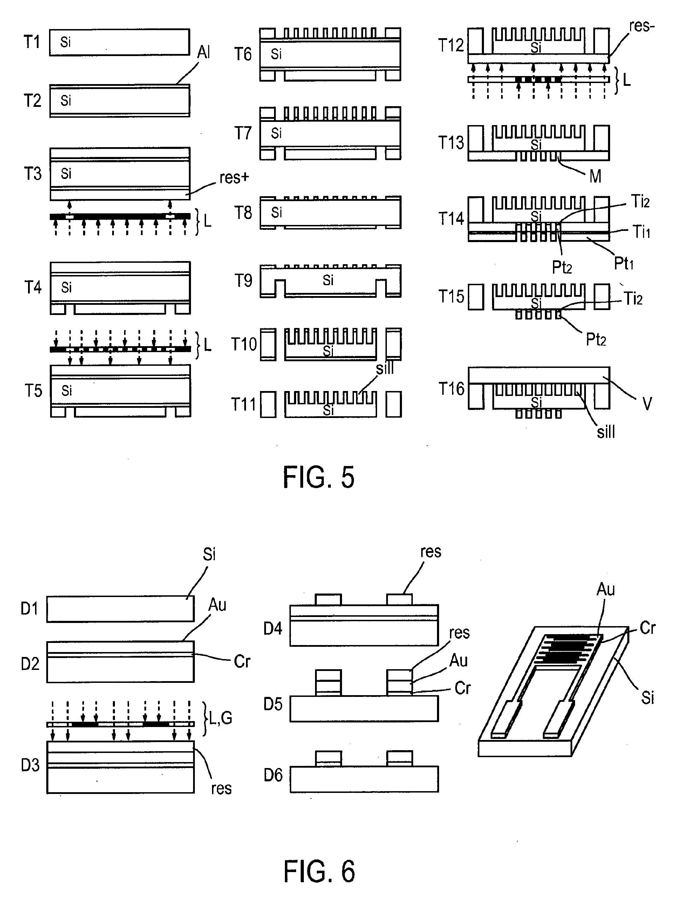

Pour les deux modules de prétraitement de l'échantillon que sont la micro-colonne et le préconcentrateur, le procédé de fabrication retenu est sensiblement le même et détaillé en Figure 5. La différence principale dans le procédé entre les deux modules concerne la profondeur de gravure des sillons. Ainsi, une profondeur de gravure de 200 μm a été définie pour les canaux formant la micro-colonne chromatographique et une profondeur de gravure de 250 μm a été définie pour les sillons de la structure de préconcentration.

Etape T1 : Nettoyage du wafer

Le substrat (Si) de la plaque (wafer) est de préférence trempé dans un bain de HF 1 % jusqu'à obtenir l'hydrophobie. Celui-ci est ensuite rincé dans de l'eau déionisée pendant 5 min. Le nettoyage consiste, dans un premier temps à dissoudre les impuretés organiques à l'aide d'acide sulfurique ($H_2SO_4$ à 150°C pendant 3 min). Dans un deuxième temps, les impuretés métalliques sont piégées par la formation d'un oxyde de surface à l'aide d'acide nitrique ($HNO_3$ pendant 3 min). Le wafer est ensuite de nouveau rincé 5 min dans l'eau déionisée, puis la couche d'oxyde formée est supprimée par un nouveau bain de HF 1 %, avant un dernier rinçage à l'eau déionisée. Le wafer est ensuite séché sous azote.

Etape T2 : Le dépôt d'Aluminium

La gravure par DRIE implique préférentiellement l'utilisation d'une couche de protection permettant de définir les zones où le substrat ne sera pas gravé. La couche de protection utilisée dans ce mode de réalisation est une couche d'aluminium -Al- de 5000 A (soit 500 nm) d'épaisseur. Le dépôt est réalisé par pulvérisation cathodique à température ambiante (24°C) dans un plasma d'argon. L'accélération des ions est obtenue par une différence de potentiel de 0.5 kV. Le dépôt est réalisé sous basse pression, à $3.10^{-7}$ Torr. La durée du dépôt dépend de l'épaisseur souhaitée d'aluminium. La vitesse de dépôt, avec ces paramètres, étant d'environ 1 000 A/min, le dépôt dure 5 min.

Les étapes suivantes consistent à définir les zones de gravure par DRIE du wafer, c'est-à-dire des zones non protégées par une couche d'aluminium. Ces étapes comportent donc une phase de photolithographie UV classique sur chaque face du wafer puis une gravure humide de la couche de l'aluminium.

Etapes T3, T4, T5 et T6 : définition des zones de gravure

L'étape T3 consiste donc à déposer sur chaque face du wafer une couche de résine photosensible res+. Dans notre cas, la résine choisie est une résine positive (PFR 7790), c'est-à-dire que les zones de résine exposées aux UV seront dissoutes. La résine est tout d'abord déposée de manière uniforme sur une face du wafer, à l'aide d'une tournette. Les paramètres d'étalement de la résine (vitesse de rotation, accélération et surtout viscosité de la résine) conditionnent l'épaisseur finale de dépôt. L'épaisseur voulue étant de 1,2 $\mu$m, la vitesse de rotation pour la résine choisie est de 4500 tr.min$^{-1}$ pour une accélération de 2000 tr.min-2 pendant 30s. L'utilisation de couche de résine sur les deux faces du wafer implique de préférence un recuit court de la résine (5 min à 110°C) afin de durcir celle-ci et d'évacuer une partie des solvants.

Le dépôt de la résine sur la deuxième face du wafer est ensuite réalisé dans les mêmes conditions suivi d'un recuit pendant 15 min à 100°C.

La première étape de photolithographie -L- sur la face arrière peut maintenant être effectuée. Les paramètres d'exposition (puissance de la lampe et temps d'exposition) permettent de définir la netteté des motifs. Dans notre cas, le temps d'insolation est fixé à 10 s avec une puissance de la lampe à 345 W.

L'étape T4 consiste ensuite à développer la résine notamment dans le but de faire apparaître les motifs d'alignement afin de pouvoir aligner le masque de la face avant, lors de l'insolation. Le wafer est donc plongé dans le bain révélateur (PRD 238) pendant 1 min 10s. Le wafer développé est ensuite rincé à l'eau déionisée pendant 3 min et séché.

L'étape T5 consiste ensuite à réaliser la photolithographie sur la face avant. Pour cela, un alignement entre les motifs du masque et ceux du wafer sur la face arrière est préférable avant l'insolation. Les coordonnées des motifs du wafer sont mémorisées et la position du masque peut ensuite être alignée sous microscope avec les coordonnées de ces motifs. Une fois l'alignement réalisé, les conditions d'exposition de la face avant sont les mêmes que pour celles de la face arrière.

L'étape T6 consiste à développer la résine dans le bain révélateur et ainsi faire apparaître les motifs de la face avant. Le wafer est plongé dans le bain jusqu'à apparition totale des motifs.

Un deuxième recuit est enfin préférable pour chauffer le wafer afin de durcir complètement la résine. Cette étape de recuit est effectuée en étuve à 100°C pendant 15 min. Le wafer est de plus nettoyé à l'aide d'un plasma 02 afin d'éliminer les résidus de résine (notamment le fond des trous graver) après le développement et améliorer la netteté des trous dans la résine.

Etape T7 : gravure de l'aluminium -Al-

La gravure de l'aluminium est réalisée dans une solution chimique composée d'acides permettant d'attaquer l'aluminium (« Al etch »). La solution est maintenue à une température de 30°C. Le contrôle de la gravure s'effectue visuellement jusqu'à élimination totale de la couche d'aluminium dans les zones délimitées par la résine. Le wafer est ensuite rincé dans l'eau déionisée.

Etape T8 : suppression de la couche de résine

Une fois la couche d'aluminium gravée, le wafer est plongé dans l'acétone afin de supprimer la couche de résine et le wafer est ensuite passé au rinceur. Le rinceur est un tambour avec un nettoyage à l'eau d'abord mis en rotation à 300 tr.min$^{-1}$, puis à 1 000 tr.min$^{-1}$ et légèrement chauffé afin de sécher le wafer.

Etapes T9 et T10 : gravure face arrière (inférieure) et face avant (supérieure)

Lors de la gravure DRIE pour la création des orifices d'accès, la gravure doit être effectuée sur la totalité de l'épaisseur du substrat -Si-, une partie étant gravée par la face avant avec les sillons, l'autre partie par la face arrière. Une sur-gravure par la face arrière (la profondeur de gravure par la face avant étant fixée par la profondeur des sillons) est préférable afin d'obtenir une paroi verticale sur l'intégralité du substrat. En effet, sans cette sur-gravure, l'intersection entre les gravures de chaque coté du substrat s'accompagnerait d'une réduction de diamètre du trou.
Pour la puce micro-colonne, la profondeur de gravure des sillons est fixée à 200 $\mu$m. L'épaisseur de substrat étant de 500 $\mu$m, la profondeur de gravure par la face arrière est donc fixée à 360 $\mu$m (dont 60 $\mu$m de sur-gravure).
Pour la puce du préconcentrateur, la profondeur de gravure des sillons est fixée à 400 $\mu$m. L'épaisseur de substrat étant de 500 $\mu$m, la profondeur de gravure par la face arrière est donc fixée à 160 $\mu$m (dont 60 $\mu$m de sur-gravure).
2 types de gaz sont injectés en alternance pour réaliser une gravure DRIE, le premier étant du SF6 pour la gravure du silicium, injecté dans l'enceinte à 300 mL.min$^{-1}$ pendant 6s, le second du C4F8 pour le dépôt du film inhibiteur, injecté dans l'enceinte à 150 mL.min$^{-1}$ pendant 2 secondes. La pression dans l'enceinte est comprise entre 3 et 4 Pa. Des cycles de 45 min de gravure sont utilisés (notamment pour éviter une chauffe trop importante du wafer). Une mesure de la profondeur de gravure après un temps donné permet de définir le temps de gravure nécessaire pour obtenir une profondeur voulue. Dans les conditions utilisées, la vitesse de gravure est généralement proche de 5 $\mu$m.min$^{-1}$.
Une fois la gravure DRIE terminée sur la face arrière, la gravure DRIE sur la face avant est réalisée dans les mêmes conditions. Les profondeurs de gravure sont respectivement fixées pour la puce micro-colonne et la puce du préconcentrateur, à 200 $\mu$m et 400 $\mu$m.

Etape T11 : Elimination de la couche d'aluminium

Suite aux étapes de gravure DRIE, le wafer est plongé dans un bain d'acide permettant d'éliminer les couches d'aluminium. La solution d'acide est maintenue à une température de 60°C, permettant ainsi d'accélérer le processus de gravure des résidus d'aluminium. Le wafer est ensuite nettoyé dans un bain d'eau oxygénée (H2O2 30 %) / acide sulfurique (H2SO4) pendant 15 min puis sous plasma 02 chaud (225°C) pour éliminer le reste des résidus.

Etapes T12, T13, T14 et T15 : Réalisation des résistances chauffantes RC1 ou RC2

Les étapes suivantes permettent ensuite la réalisation des résistances chauffantes en titane / platine. Le procédé utilisé pour le dépôt des résistances est un procédé « lift-off », c'est-à-dire une technique additive (en opposition aux techniques de gravure) utilisant une couche sacrificielle.
Les étapes T12 et T13 constituent une étape de photolithographie -L-classique avec l'utilisation d'une résine négative « res- » (la résine non exposée est dissoute) comme couche sacrificielle. La résine est donc étalée à l'aide d'une tournette afin d'obtenir une épaisseur de 7 $\mu$m. Un recuit à 110°C pendant 90 secondes est ensuite réalisé. Un alignement sous microscope du masque M contenant les motifs des résistances est réalisé après mémorisation de la position des motifs d'alignements gravés sur le wafer. La résine est ensuite insolée pendant 90 s avant un nouveau recuit de 90 s à 110°C. Le wafer est ensuite plongé dans le bain révélateur (AZ351 B / eau) puis passé au rinceur.
L'étape T14 consiste à réaliser les dépôts successifs des deux couches de métaux utilisées pour la réalisation des résistances, soit 500 A (50 nm) de titane -Ti1- pour la couche d'accroche et 1 000 A (100 nm) de platine -Pt1-. Avant le dépôt des couches de métaux, un décapage du substrat au plasma d'argon est réalisé afin de nettoyer correctement les trous réalisés et éviter les résidus de résine dans le fond. Les dépôts des couches de titane -Ti- et de platine -Pt- sont réalisés sous basse pression (1.10-2 mbar) respectivement pendant 1 min 15 s et 1 min 35 s sur toute la surface du wafer atteignant le substrat dans les zones gravées et se déposant sur la résine dans les zones non gravées.
L'étape T15 consiste ensuite à supprimer la couche sacrificielle. Pour cela, le wafer est plongé dans un bain d'acétone sous ultrason. Lors de la suppression de la couche sacrificielle, les couches de métaux en contact sur la résine sont arrachées. Après suppression de la couche sacrificielle, les couches de métaux (Ti2 et Pt2) restent uniquement dans les zones de contact avec le substrat. Le wafer subit ensuite différents nettoyages pour éliminer les différents résidus (bain pour les résidus de polymères comme les résines, bain eau oxygénée / acide sulfurique et bain de HF 1 %).

Etape T16 : soudure anodique

Le micro-usinage direct du silicium permet d'obtenir des structures ouvertes sur l'extérieur (sillons -sill-). La soudure anodique entre wafer est une technique qui permet de souder ensemble des substrats de silicium ou de matériaux différents (tel que le verre) afin d'obtenir des cavités closes. La solution retenue pour la réalisation de cavités closes est une soudure anodique entre le wafer de silicium (contenant les sillons) et un wafer en verre -v-.

La soudure anodique s'effectue à une température élevée de 420°C sous vide (10-4 à 10-5 mbar) et sous un champ électrique de 500 V pendant 10 min.

7.1.D Connexions microfluidiques

Pour permettre le remplissage des microstructures mais également la circulation des échantillons d'air, celles-ci doivent être équipées des connecteurs microfluidiques (NanoPorts) présentés précédemment.

Le procédé de mise en place des connecteurs implique de préférence dans un premier temps un nettoyage à l'éthanol de la surface du wafer afin d'assurer une bonne adhésion de la colle. Une fois la surface nettoyée, l'anneau de colle est positionné autour de l'orifice d'accès de la microstructure. Le joint d'étanchéité est ensuite installé sous le corps du connecteur dans une « bague » de positionnement. Le corps du connecteur est ensuite déposé sur l'anneau de colle et doit soigneusement être positionné pour permettre l'alignement des orifices du wafer et du connecteur. Une vérification de l'alignement est effectuée visuellement.

Une fois le connecteur positionné, le wafer et le corps du connecteur sont maintenus en place à l'aide d'une pince permettant ainsi la compression du joint et assurant l'étanchéité de la connexion. Une plaque de verre est préalablement placée en contact sur l'autre face du wafer (coté verre) pour éviter un contact direct entre la microstructure et la pince. L'ensemble est ensuite placé au four à 180°C pendant 2 heures.

7.2 PROCEDE DE FONCTIONNALISATION DES MODULES

7.2.A Microstructure de séparation

Une fois les micro-colonnes réalisées et les connexions microfluidiques en place, celles-ci ont été fonctionnalisées avec leur remplissage par une phase stationnaire permettant l'élution et la séparation des composés gazeux.

Lors de l'analyse des COV pour le développement des indices de contamination, la séparation des différents composés a été réalisée par chromatographie en phase gazeuse à l'aide d'une colonne capillaire contenant du PDMS (de préférence 5 % phényl - 95 % dimethylpolysiloxane) comme phase stationnaire. Le choix d'une composition de colonne similaire, avec une phase stationnaire constituée de PDMS (Polydiméthylsiloxane), a été retenu pour la réalisation des micro-colonnes.

Le PDMS utilisé (Sylgard® 184, commercialisé par la société Dow corning) est commercialisé sous forme de deux liquides, la base et le réticulant. Les deux constituants sont généralement mélangés avec un rapport massique de 10:1 (base:réticulant). Les réactions de réticulation commencent avec le mélange, induisant donc une augmentation progressive de la viscosité, suivie de la formation d'un gel. Lors du mélange, des bulles d'air sont introduites et doivent être évacuées avant de procéder au remplissage afin de permettre un dépôt uniforme sur toute la longueur de la colonne. Afin d'évacuer les bulles, le mélange est placé dans une chambre sous vide. Le temps d'évacuation varie selon la quantité d'air introduite.

Afin d'être inséré dans le micromodule et de permettre la réalisation du canal avec la substance active, à savoir la phase stationnaire, le mélange est ensuite dilué dans un solvant. Pour obtenir la meilleure efficacité possible de séparation avec une colonne chromatographique, le dépôt d'une couche homogène de phase stationnaire est nécessaire. Le dépôt de la couche stationnaire peut alors être réalisé suivant deux méthodes, le remplissage dynamique (« dynamic coating ») ou statique (« static coating ») :

- La procédure de remplissage dynamique consiste à remplir de solution une partie de la colonne qui est ensuite poussé à travers la colonne, à une vitesse approximative de 1 à 2 cm.s$^{-1}$, par pression d'un gaz inerte. Une fine couche de la solution est alors laissée sur les parois de la colonne. Après remplissage, le flux de gaz est maintenu pendant l'évaporation du solvant, laissant ainsi une couche de phase stationnaire sur les parois de la colonne. La colonne est chauffée au dessus du point d'ébullition du solvant afin d'éliminer les traces résiduelles de solvant. L'épaisseur du film est définie par proportion de solvant utilisé pour la dilution de la phase. Cette méthode simple pose cependant des problèmes conduisant à un dépôt non-uniforme de la phase stationnaire (Xu et Vermeulen, 1988).

- La procédure de remplissage statique consiste à remplir entièrement la colonne avec la solution de phase stationnaire diluée dans le solvant. Après remplissage de la colonne avec la solution, une extrémité de la colonne est bouchée et l'autre extrémité est reliée à une pompe à vide. La colonne est ensuite placée au four ou dans un bain-marie afin de contrôler la température de la colonne. L'évaporation du solvant s'effectue par application d'un vide, laissant un dépôt uniforme de phase stationnaire sur les parois de la colonne. Avec cette procédure, le ratio entre le solvant et la phase stationnaire est connu précisément et donc, en connaissant la densité de la phase stationnaire, l'épaisseur du dépôt peut être déterminée avec précision (Xu et Vermeulen, 1988).

Le choix d'un remplissage statique a été retenu pour la fonctionnalisation des micro-colonnes. Le processus de fonctionnalisation des colonnes comporte donc 3 étapes :

Etape 1 : Préparation de la solution

Avec le procédé de remplissage statique, l'épaisseur de la couche de phase stationnaire dépend de la concentration de la solution et peut être déterminée par l'équation suivante dans le cas d'une colonne capillaire de section circulaire:

$$\frac{1}{4}\pi d_c^2 c = \pi d_c d_f$$

Où dc est le diamètre interne de la colonne, c la concentration de la solution, et df l'épaisseur de la couche déposée, d'où :

$$c = 4\frac{d_f}{d_c}$$

Le solvant retenu pour le remplissage de la micro-colonne est le n-pentane. Ce solvant présente l'avantage d'être très volatil puisque sa température d'ébullition est de 36,06°C facilitant ainsi son évaporation. De plus, il est aprotique, ce qui signifie qu'il ne possède pas d'hydrogène acide (hydrogène lié à un hétéroatome tel que l'atome d'oxygène ou d'azote) susceptible de réagir avec les fonctions siloxanes (fonctions Si-O-Si du PDMS). Des solvants protiques comme l'eau ou l'éthanol conduisent à une gélification du polydiméthylsiloxane.

La micro-colonne utilisée pour les essais est de section rectangulaire. Sachant que l'épaisseur souhaitée de PDMS est de 200 nm et que la micro-colonne fait 200 $\mu$m de profondeur et 150 $\mu$m de large, le rapport $V_{PPMS}/V_{pentane}$ a été estimé à 0,47 %.

Afin de comparer la concentration de la solution de PDMS utilisée pour la micro-colonne par rapport à celle couramment utilisée dans les colonnes capillaires, un calcul a été réalisé en approximant la valeur du diamètre interne à celle de la profondeur de la micro-colonne dans l'équation ci-dessus. Pour une profondeur de 200 $\mu$m, le rapport $V_{PDMS}/V_{pentane}$ a été estimé à 0,4 %. Cette valeur est donc cohérente avec celle calculée précédemment.

Afin de préparer la solution, ce rapport de volume est converti en un rapport de masse : Sachant que p = m / V où p est la masse volumique du composé, m la masse et V le volume, le rapport de masse équivaut à :

$$\frac{m_{PDMS}}{m_{pentane}} = \frac{\rho_{PDMS}}{\rho_{pentane}} \times \frac{V_{PDMS}}{V_{pentane}} = \frac{1,1}{0,63} \times 0,0047 = 0,0082$$

Le rapport de masse entre le PDMS et le pentane est donc de 0,82 %.

Etape 2 : Remplissage de la colonne

Cette étape consiste à remplir entièrement la micro-colonne de solution. Ainsi la solution de PDMS diluée dans le solvant est injectée dans la micro-colonne à l'aide d'un pousse seringue. Des vannes sont installées à chaque extrémité de la colonne afin de pouvoir contrôler facilement l'ouverture et la fermeture du canal.

Lors du remplissage, les deux vannes sont ouvertes et la solution circule donc à travers la colonne. Une fois la colonne complètement remplie, les vannes de sortie puis d'entrée sont fermées successivement. La colonne peut ainsi être déconnectée du pousse-seringue. Avant la connexion de la colonne à la pompe à vide pour l'étape d'évaporation du solvant, le tube d'accès en entrée de la microstructure est préalablement déconnecté afin d'évacuer la solution contenue à l'intérieur du tube. La formation d'un ménisque est observée en entrée de la microstructure. Ce ménisque correspond à la surface de la solution (PDMS dilué dans le pentane) où se produit l'évaporation du solvant. Celui-ci permettra donc par la suite de contrôler l'évaporation du solvant.

La concentration de la solution joue également un rôle important dans cette étape du procédé. En effet, dans le cas d'une dilution trop faible du PDMS dans le solvant, la paroi du ménisque forme une membrane ne permettant pas l'évaporation du solvant.

Etape 3 : Evaporation du solvant et fonctionnalisation

Une fois le tube d'accès en entrée de la colonne reconnecté, la pompe à vide peut être connectée à la colonne. Un raccord en T est inséré entre la vanne d'entrée et la pompe afin de créer une fuite contrôlée par une bague permettant ainsi de contrôler le vide appliqué dans la colonne. L'étanchéité au niveau des connexions entre les éléments du montage est un paramètre important afin d'assurer la continuité lors du processus d'évaporation du solvant.

Une fois connectée, la micro-colonne est alors mise en contact sur une plaque chauffante régulée en température permettant de contrôler la température appliquée à la microstructure.

Lors de la mise en contact de la microstructure un léger déplacement du ménisque est observé suite à la dilatation du solvant due au changement de température. En effet, lors d'un procédé de remplissage statique, la chaleur extérieure est transférée au ménisque ce qui permet l'évaporation du solvant. La vapeur du solvant ainsi créée, ayant une pression supérieure à celle de l'orifice de la colonne, traverse la colonne vers la sortie.

Dans ce procédé, les deux principaux facteurs sont le transfert de masse (vapeur de solvant) vers la sortie et le transfert de chaleur dans la colonne. De la loi de Poiseuille découle l'équation qui régit ce phénomène physique :

$$\frac{dV}{dt} = \frac{\pi d_c^4 (P^2 - P_0^2)}{256 L \eta_v P_{atm}}$$

Où $dV/dt$ est le débit d'évaporation du solvant, P et $P_0$ sont respectivement les pressions au niveau du ménisque et à la sortie de la colonne, $\eta_v$ la viscosité de la vapeur du solvant, L la distance entre le ménisque et la sortie de la colonne, $P_{atm}$ la pression atmosphérique et dc le diamètre interne de la colonne.

Cette équation permet de mettre en évidence que le débit d'évaporation du solvant à travers la colonne est proportionnel au terme $P_2 - P_0^2$ et inversement proportionnel à L. De l'équation précédente est déduite l'équation régissant la vitesse de remplissage :

$$\frac{dL}{dt} = \frac{273,16 d_c^2 (P^2 - P_0^2) M}{64 \cdot 22400 T_c L \eta_v d_1 P_{atm}}$$

Où $dL/dt$ est la vitesse de remplissage, M la masse molaire du solvant, Tc la température de remplissage et d1 la densité du solvant.

Dans un procédé de remplissage statique conventionnel, P est généralement faible et par conséquent la vitesse de remplissage $dL/dt$ est également faible, en particulier quand la colonne à remplir est longue ou que son diamètre interne est faible. Lors de l'utilisation d'une température de remplissage élevée, le terme $P_2 - P_0^2$ peut être grand malgré le fait que $P_0$ soit grand également. Cependant, l'utilisation de température élevée entraine une différence entre la tension de vapeur $P_1$, pression d'équilibre entre la phase liquide et la phase vapeur de la solution, et P la pression au niveau du ménisque qui sera plus élevée. Ce phénomène risque d'augmenter la formation de bulles conduisant à une fonctionnalisation de la colonne inhomogène.

Afin d'augmenter la vitesse de remplissage, une pompe à vide est connectée en sortie de la colonne. Celle-ci permet de diminuer la valeur de $P_0$ favorisant ainsi le transfert de masse.

Les conditions initiales du procédé d'évaporation sont donc les suivantes : la pompe à vide (éteinte) est connectée à la vanne d'entrée (fermée) avec l'insertion de la bague de contrôle. La vanne de sortie est également fermée. Dans un premier temps, la pompe est allumée avec la bague de contrôle ouverte afin de créer une fuite. La vanne d'entrée est ensuite ouverte et la bague de contrôle fermée progressivement afin de créer un vide progressif dans la colonne. Une mesure du vide est réalisée en amont de la vanne d'entrée.

Dans notre cas, l'évaporation du solvant débute pour un vide d'environ 0,2 bars et la température de la colonne est maintenue à 33°C. La progression du ménisque, formé par l'évaporation du solvant, permet de suivre la progression du dépôt. Le ménisque se déplace donc dans la colonne de l'entrée vers la sortie.

Le procédé d'évaporation dure environ 15 min soit une vitesse d'environ 0,5 cm.s$^{-1}$. Une fois le ménisque arrivé en sortie de la colonne, le vide est maintenu pendant quelques minutes afin de permettre l'évaporation du solvant contenu dans le tube de sortie. Le vide est ensuite progressivement cassé à l'aide de la bague de contrôle.

La dernière étape pour fonctionnaliser la micro-colonne consiste à placer la colonne dans un four à 80°C pendant 2 heures afin de réticuler la couche de PDMS déposée sur les parois.

Le réglage des paramètres environnementaux que sont la température et le vide appliqué à la micro-colonne dans le processus d'évaporation du solvant est délicat à obtenir. En effet, une température trop faible (inférieur à 31 °C) ne permet pas l'évaporation du solvant alors qu'une température trop élevée (supérieur 35°C, soit environ la température d'ébullition du solvant) entraîne une discontinuité de l'évolution du processus d'évaporation à travers la colonne. Le choix d'une température de 33°C a donc été retenu, cependant le processus d'évaporation reste sensible à la température.

L'utilisation d'un vide contrôlé permet de pallier ce problème. En effet, si trouver la température adéquate reste délicat, une fois la température de la microstructure stabilisée, la gestion du vide appliqué dans la colonne permet de contrôler la vitesse d'évaporation du solvant. Une augmentation progressive du vide permet ainsi d'une part de débuter le processus d'évaporation (0,2 bars dans ce mode de réalisation) et d'autre part d'en contrôler la vitesse (plus le vide est important, plus la vitesse d'évaporation augmente).

7.3.B Microstructure de concentration.

Le développement des indices de contamination fongique a permis d'identifier le Tenax TA -Tx- comme un adsorbant pertinent pour piéger les molécules sélectionnées pour la détection fongique. Ce matériau est disponible sous forme de grains dont la taille a été sélectionnée à l'aide de tamis pour obtenir des diamètres compris entre 50 et 100 μm.

Le matériau étant sous forme de grains, celui-ci est donc dilué dans un solvant afin de pouvoir être introduit dans la microstructure à l'aide d'un pousse-seringue. Le procédé de remplissage pour la microstructure de concentration est moins complexe que pour la fonctionnalisation de la micro-colonne. Néanmoins, une phase d'optimisation de la fonctionnalisation de la microstructure par l'insertion des grains de Tenax à l'intérieur du dispositif est préférable. Les paramètres de dilution et de débit d'insertion sont notamment à définir afin d'éviter des risques de colmatage du canal d'accès à la structure ou de destruction de la microstructure.

Le solvant retenu pour la fonctionnalisation de la microstructure de concentration est l'éthanol. Pour le paramètre de dilution, le rapport entre le volume de Tenax et le volume d'éthanol ($V_{Tenax}/V_{Ethanol}$) est estimé entre 1 et 2 %.

Le processus de remplissage de la microstructure de concentration consiste donc à injecter via le pousse-seringue les grains de Tenax dilués dans l'éthanol. Un aimant, inséré dans la seringue, couplé au micro-agitateur permet de maintenir en suspension les grains dans le solvant. Ceci permet de pallier le problème de sédimentation des grains de Tenax dans le fond de la seringue. Les grains transportés par le solvant sont ainsi injectés les uns après les autres dans la structure. La microstructure, en position verticale, permet aux grains de s'écouler dans la structure vers la sortie. La présence de la grille constituée des micropiliers en sortie de la structure permet alors de filtrer les grains.

L'utilisation d'un pousse seringue permet de contrôler le débit de remplissage et donc de réguler l'injection des grains dans la structure via le canal d'accès. Un débit de remplissage trop important peut cependant conduire à la destruction partielle des murs formant les canaux ou encore à la formation d'une fissure sur la face arrière de la structure.

Le choix d'un débit de 250 μL.min$^{-1}$ pour le remplissage de la microstructure a été retenu. Celui-ci permet d'éviter, d'une part le colmatage du canal d'accès par les grains injectés dans la structure, d'autre part, de ne pas soumettre la microstructure à de trop fortes contraintes, notamment en fin du remplissage de la structure.

Une fois la microstructure complètement remplie, celle-ci est placée au four à 100°C pendant 2 heures afin d'éliminer les traces résiduelles de solvant, puis conditionnée pendant deux heures à 140°C avec un passage d'air filtré à travers la structure.

7.4 PROCEDE DE FABRICATION DU MODULE DE DETECTION

Le système d'acquisition de données, utilisé lors des tests sur les capteurs polymères conducteurs, ne permet pas une intégration pour la réalisation d'une microchaîne d'analyse des COV d'origine fongique. Dans cette optique, un module de détection, basé sur la matrice de polymères composant le coeur du système d'acquisition, a été réalisé. Ainsi, le module de détection se compose de 4 paires d'électrodes interdigitées pour les dépôts des couches polymères, et d'une chambre en acier inoxydable permettant le confinement des couches polymères et le passage de l'air.

7.4.A Electrodes interdigitées

La carte utilisée précédemment dans le système d'acquisition, ne permet pas de réaliser simplement et précisément des dépôts.

D'une part, sa configuration et la méthode de dépôt des différents polymères imposent de déposer le même polymère sur toutes les électrodes. En effet, des dépôts successifs d'autres polymères ou électrolytes ainsi que le dopage chimique (vapeur de diiode) altèrent les dépôts précédents et ne permettent donc pas de comprendre le mécanisme d'interaction uniquement entre le COV et le polymère. D'autre part, la configuration des électrodes utilisées ne permet pas un contrôle de la zone de dépôt du polymère entre les deux électrodes.

Des « puces » indépendantes constituées d'une unique paire d'électrodes interdigitées ont ainsi été développées pour pallier ces inconvénients. Comme pour la carte précédente, les électrodes sont réalisées en or avec une couche d'accroche en chrome. Le procédé de réalisation est présenté en Figure 6.

Etape D1 : Nettoyage du wafer

Le nettoyage est identique à celui utilisé pour la réalisation des précédents modules.

Etape D2 : Le dépôt des couches de chrome et d'or

Pour la réalisation des électrodes, le procédé consiste à déposer successivement de manière homogène la couche d'accroche en chrome sur une épaisseur de 500 A (durée de dépôt de 1 min 15s), puis la couche d'or sur une épaisseur de 10 000 A (durée de dépôt 10 min). Les dépôts sont réalisés par pulvérisation cathodique à température ambiante (24°C) dans un plasma d'argon.

Les étapes suivantes consistent à définir les zones de gravure pour la réalisation des motifs. Ces étapes comportent donc une phase de photolithographie UV classique puis une gravure humide des couches de chrome et d'or.

Etapes D3 et D4 : définition des zones de gravure

Une fois les couches déposées, le procédé consiste à réaliser une étape de photolithographie classique permettant de réaliser des zones interdigitées.

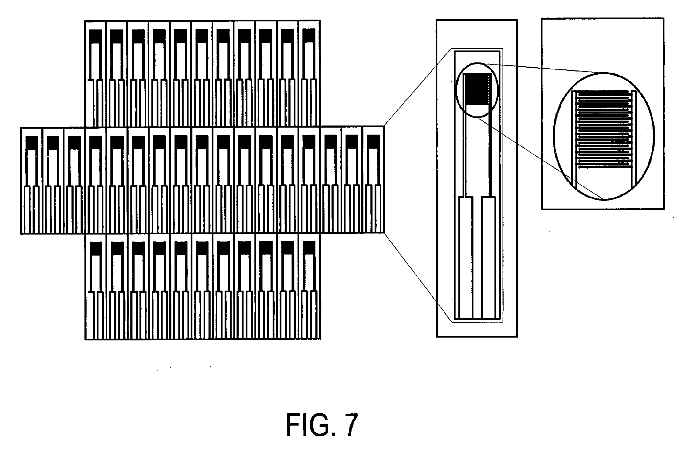

L'étape D3 consiste donc à déposer une couche de résine photosensible (résine positive PFR 7 790) à l'aide d'une tournette. L'épaisseur voulue est de 1,2 $\mu$m (vitesse de rotation de 4 500 tr.min$^{-1}$, accélération de 2 000 tr.min$^{-2}$ pendant 30 s). Le recuit de la résine est réalisé sur plaque chauffante (« hot plate ») à 110°C pendant 3 min. L'étape de photolithographie est ensuite effectuée avec un temps d'insolation de la résine fixé à 10 s et une puissance de la lampe de 345W. Une image du masque utilisé lors de l'insolation est présentée en Figure 7. Sur cette figure sont représentés de gauche à droite, un ensemble de puces puis un puce et enfin une électrode interdigitée.

Les caractéristiques géométriques retenues pour la réalisation des puces contenant une paire d'électrodes interdigitées sont reportés dans le tableau 10.

Tableau 10 : Caractéristiques géométriques (en $\mu$m) des électrodes

| Carie | | Contact | | | Electrodes interdigitées | | | |
|---|---|---|---|---|---|---|---|---|
| longueur | largeur | longuer | largeur | pas | longueur | largeur | pas | nombre |
| 26000 | 5080 | 12000 | 1580 | 2540 | 2500 | 60 | 240 | 13 |

L'étape D4 consiste ensuite à développer la résine formant ainsi les motifs gravés ultérieurement. Le wafer est donc plongé dans le bain révélateur (PRD 238) pendant 1 min 10 s. Le wafer développé est ensuite rincé à l'eau déionisée pendant 3 min et séché avant le deuxième recuit post développement, permettant ainsi de durcir complètement la résine. Cette étape de recuit est effectuée en étuve à 100°C pendant 15 min. Le wafer est de plus nettoyé à l'aide d'un plasma 02 afin d'éliminer les résidus de résine (notamment le fond des trous gravés) après le développement et améliorer la netteté des trous dans la résine.

Etape D5 : gravure des couches de chrome et d'or

Les gravures de l'or puis du chrome sont réalisées dans des solutions chimiques composées d'acides. Les solutions sont maintenues à une température de 30°C.

Le premier bain est réalisé dans une solution permettant la gravure de la couche d'or. Le contrôle de la gravure s'effectue visuellement jusqu'à élimination totale de la couche d'or dans les zones délimitées par la résine. Le wafer est ensuite rincé dans l'eau déionisée.

Le deuxième bain est réalisé dans une solution permettant la gravure de la couche de chrome. Le contrôle de la gravure s'effectue visuellement jusqu'à élimination totale de la couche de chrome dans les zones délimitée par la résine. Le wafer est ensuite rincé dans l'eau déionisée.

Un nouveau bain très rapide est ensuite réalisé pour la gravure de l'or et du chrome à l'interface des deux couches. Le wafer est ensuite rincé dans l'eau déionisée.

Etape D6 : suppression de la couche de résine

Une fois les couches gravées, le wafer est plongé dans l'acétone afin de supprimer la couche de résine restante et le wafer est ensuite passé au rinceur.

Une fois le procédé de fabrication de cartes terminé, une couche de résine d'épaisseur 3 $\mu$m est de nouveau déposée sur toute la surface du wafer afin d'éviter des dépôts de particule sur les électrodes lors de la découpe des différentes cartes présentes sur le wafer. La résine est ensuite recuite à 110°C pendant 1 min 30 s sur plaque chauffante. Avant d'utiliser les cartes pour les fonctionnaliser avec un polymère conducteur, un bain dans l'acétone permet l'élimination de la couche de protection en résine.

7.4.B Chambre de confinement

Comme pour le système de test, l'utilisation des capteurs polymères implique de préférence le développement d'une chambre permettant le confinement des couches sensibles afin de les exposer uniquement aux échantillons d'air circulant dans le système. Le choix de l'acier inoxydable comme matériau pour la réalisation de cette chambre et l'utilisation de joint en téflon (PTFE) permet de limiter la génération d'un bruit de fond, l'acier inoxydable et le téflon étant des matériaux non émissifs dans les conditions expérimentales de ce mode de réalisation.

La chambre de confinement se divise par exemple en deux parties. Une partie socle dans laquelle sont creusées quatre sillons permettant l'insertion des électrodes. Une butée permet de placer la zone de dépôt du polymère présent sur l'électrode en face des « volumes » de confinement présent sur la partie capot.

La partie capot est donc constituée de quatre volumes de confinement liés entre eux par un canal. Ces volumes de confinement permettent donc d'englober uniquement la zone de dépôt des polymères et ainsi de limiter la dilution des échantillons dans des volumes trop importants. Afin d'assurer l'étanchéité entre l'électrode et les volumes de confinement, un joint téflon est inséré dans une gorge entre les deux éléments. Le joint présente ainsi quatre ouvertures permettant d'exposer les échantillons d'air présent dans les volumes de confinement aux couches sensibles des électrodes.

Enfin, la circulation des échantillons d'air est assurée en entrée et en sortie de la chambre sur la partie capot à l'aide de NanoPort.

Les dimensions et l'espace entre chaque capteur a été défini afin de pouvoir utiliser un connecteur encartable avec un pas de 2,54 mm. Ce connecteur permet ainsi de réaliser la liaison entre les capteurs et une carte de traitement de l'information.

7.4.C Traitement de l'information

Les capteurs fonctionnalisés avec un polymère conducteur sont placés dans la chambre de confinement afin de pouvoir être utilisé comme module de détection du système d'analyse. Le principe de fonctionnement de ces capteurs est basé sur la variation de conductivité du polymère induite par l'adsorption de composés gazeux à sa surface. Cette adsorption dépend de l'affinité du composé avec le site actif présent dans le polymère.

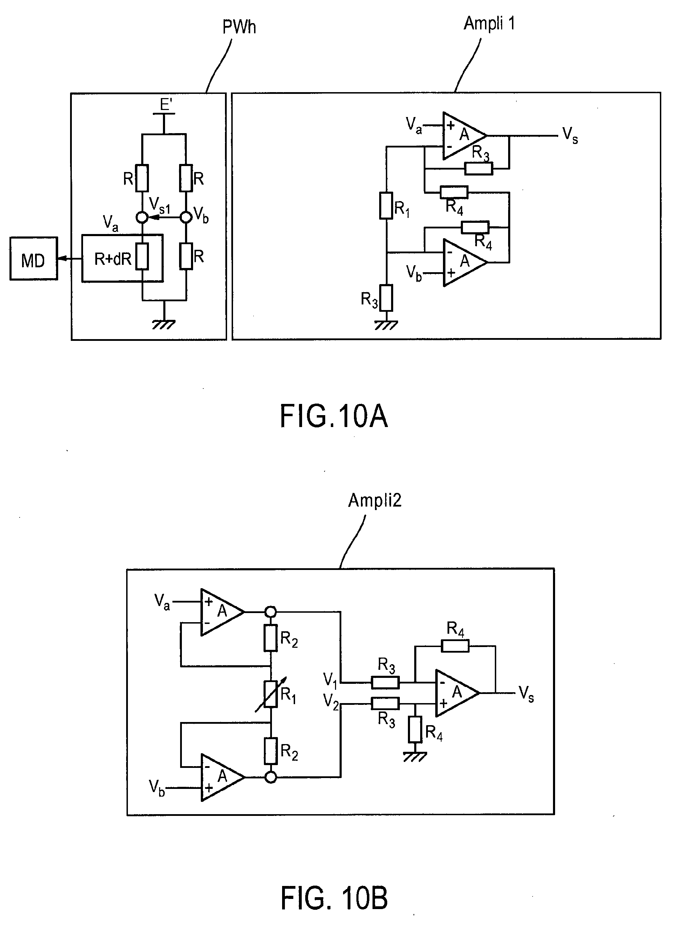

Afin de convertir cette variation de conductivité en un signal mesurable, le capteur est placé dans un montage permettant de mesurer une variation de résistance électrique nommé « Pont de Wheatstone » -P Wh-. Le principe de ce montage consiste à équilibrer les deux branches du pont en plaçant le capteur dans une des deux branches. Les variations de résistivité du capteur engendrent un déséquilibre et l'apparition d'une tension entre les deux branches du pont (voir le schéma figures 10A et 10B).

Dans ce montage, dR représente la variation de résistivité du capteur lors de l'adsorption de composés gazeux à sa surface. La valeur de la tension de sortie VS1 est définie par les éléments du montage et vaut :

$$V_{S1} = \frac{VCC}{2}\frac{dR}{R}$$

Où VCC est la tension appliquée au montage Pont de Wheatstone -P Wh-.

Lorsque toutes les résistances sont égales (dR = 0), la valeur de VS1 est égale 0. Lorsque la résistivité du capteur varie (dR ≠ 0), alors VS1 est l'image de cette variation et peut-être amplifié à l'aide d'un montage amplificateur à base d'amplificateur opérationnel -Ampli 1- (voir le schéma figure 10A).

Ce montage permet d'amplifier la tension comprise entre Va et $V_b$ (soit $V_{S1}$) en fonction de la valeur des résistances composant le montage :

$$V_s = (V_a - V_b)G$$

et

$$G = 4 + \frac{60000}{R_1}$$

avec $R_3$ = 30kΩ et $R_4$ = 10kΩ

La tension de sortie du montage représentant les variations de résistivité du capteur devient alors :

$$V_s = V_{s1}G = \frac{dR}{R}\frac{VCC}{2}\left(4 + \frac{60000}{R_1}\right)$$

Dans cette optique, une carte permettant de traiter les informations transmises par les capteurs a été développée. Elle consiste à associer à chaque capteur, un pont de Wheatstone pour convertir la variation de résistivité en tension et un montage amplificateur -Ampli 1- (Figure 10A). A titre d'alternative, le montage d'amplification -Ampli 2- de la figure 10B peut être utilisé sans sortir du cadre de l'invention.

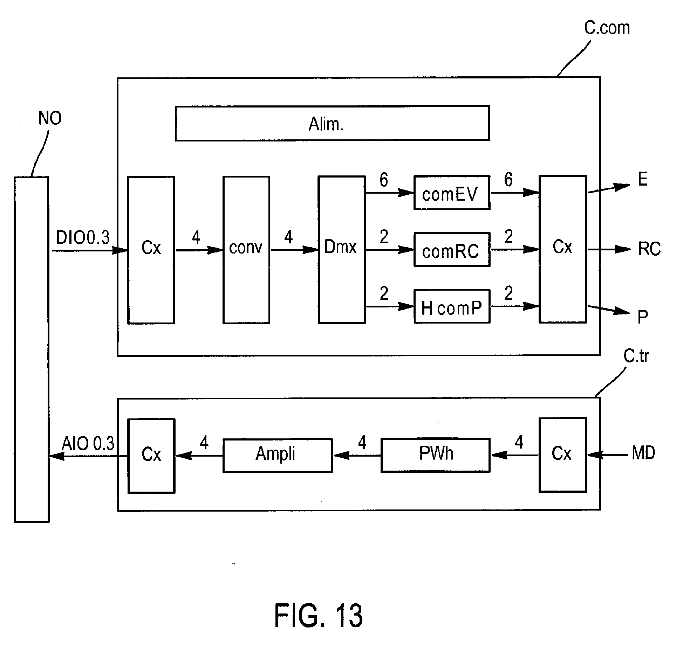

Le schéma du montage développé sous Cadence Allegro Design Entry représente un des quatre montages utilisés pour le traitement de chacun des capteurs. Un bornier regroupe les signaux de mesure des différents capteurs et permet la connexion avec un noeud de mesure pour permettre à l'utilisateur de récupérer les informations transmises. Les références Cx (figure 13) désignent les borniers de connexion de la carte de traitement et de la carte de commande.

L'ensemble des quatre montages et du bornier forme ainsi une carte de traitement des signaux des différents capteurs. Cet ensemble peut alors être intégré à l'aide de l'outil cadence Allegro PCB Design pour développer la carte. Cet outil permet le placement et le routage entre les différents composants de la carte en intégrant toutes les liaisons définies sous Cadence Allegro Design Entry. La carte de traitement des signaux transmis par les capteurs peut alors être réalisée.

## 7.5 CARACTERISATION DES MODULES

### 7.5.A Module de concentration de l'échantillon

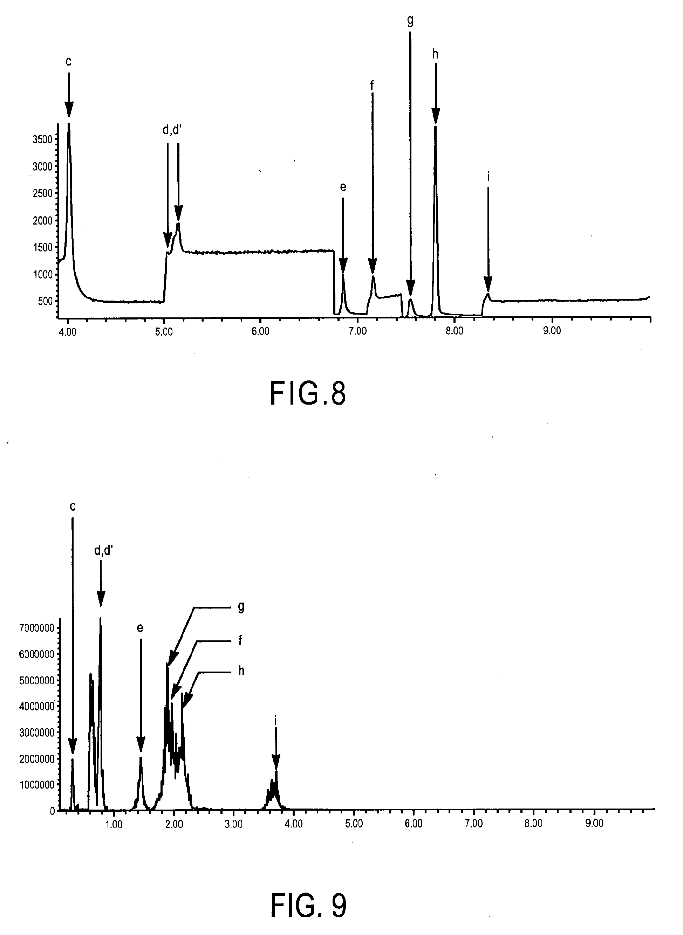

En se référant à la figure 8, le piégeage et la désorption de 8 traceurs ont été validés en chromatographie classique. Les 8 traceurs ont été retrouvés avec les ambiances contaminées par des moisissures, mais les concentrations observées sont plus faibles.

Les pics de la figure 8 sont les suivants :

| | | |
|---|---|---|
| c :méthylfurane ; | d :2-méthyl-1-butanol ; | d' :3-méthyl-1-butanol ; |
| e :4-heptanone ; | f :3-heptanol ; | g :méthoxybenzène ; |
| h :α-pinène ; | i :1-octèn-3-ol. | |

### 7.5.B Module de séparation de l'échantillon

Le module de séparation a également été validé par chromatographie et spectrométrie de masse. Le chromatogramme obtenu à partir des 8 traceurs en solution est présenté figure 9.

La micro-colonne testée a permis la séparation de 7 traceurs parmi les 8 testés: le 3-méthyl-1-butanol et le 2-méthyl-1-butanol, qui sont des isomères, sont coélués.

L'ensemble de ces essais a également permis de trouver les conditions les plus adaptées pour la séparation des traceurs (isotherme à 40°C et débit d'hélium à 0,5 mL.min⁻¹) et de réduire considérablement le temps d'analyse (10 min environ pour les micro-colonnes contre 1 h 30 s pour une colonne standard). Ainsi, ces essais ont permis de valider le module de séparation en montrant l'obtention de temps de rétention différents pour 11 des traceurs permettant le calcul de l'indice de contamination fongique.

7.5.C Module de détection

Des essais ont été réalisés sur le polypyrrole/octane sulfonate de sodium (0,3 M) et le PEDOT-PSS. Ces deux couches sensibles ont été exposées à différents Composés Organiques Volatiles (COV). Ces essais ont été réalisés selon les montages des figures 10A et 10B.

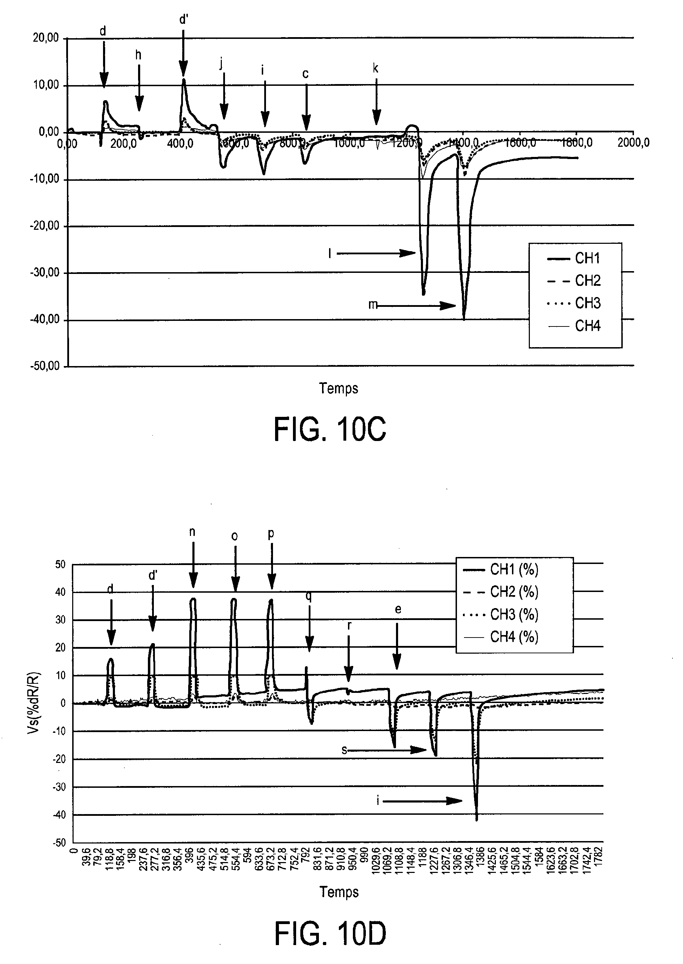

Lors de l'essai présenté en figure 10C, les 2 polymères conducteurs électroniques sont exposés à huit COV issus de l'indice de contamination fongique, à de l'eau distillée (l)et de l'éthanol(m). De manière générale, les références CH1 à CH4 désignent différents essais.

Des différences fractionnelles de résistance négatives ont été observées pour six COV (alpha-pinène, anisole (j), 1-octèn-3-ol, 2-méthylfurane, 3-heptanol(k)), l'éthanol et l'eau. Cependant, des différences fractionnelles de résistance positives ont été observées pour deux COV, le 2-méthyl-1-butanol et le 3-méthyl-1-butanol. Ces réponses sont similaires pour ces 2 COV isomères qui sont des alcools primaires. Lorsque les polymères sont exposés à l'éthanol qui est un alcool primaire, une différence fractionnelle de résistance négative a été observée. Ceci est expliqué par le fait qu'il contient de l'eau.

Afin de vérifier la sélectivité à des alcools primaires, les deux couches sensibles ont ensuite été exposées à une série d'alcools primaires, 2-méthyl-1-butanol, 3-méthyl-1-butanol, 1-butanol (n), 1-pentanol (o) et 1-hexanol (p), à deux alcanes, l'heptane (q) et l'octane (s), ainsi qu'au 3-heptanol (r), au 1-octèn-3-ol et à la 4-heptanone. Des différences fractionnelles de résistance positives ont été observées pour les alcools primaires. Cependant, des différences fractionnelles de résistance négatives ont été observées pour les autres COV. Les résultats sont illustrés en figure 10D. Sur cette figure, les références CH1 et CH3 désignent des essais avec du PEDOT/PSS, et les références CH2 et CH4, des essais avec de l'OSS (0,3M).

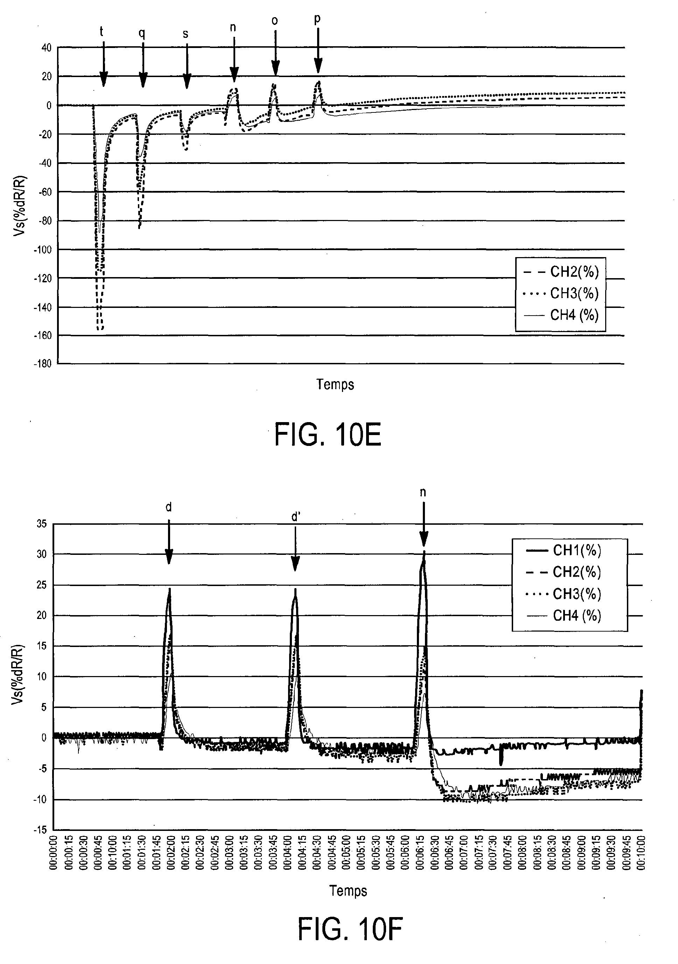

Le PEDOT-PSS a ensuite été exposé à une série d'alcools primaires, 2-méthyl-1-butanol, 3-méthyl-1-butanol, 1-butanol, 1-pentanol et 1-hexanol, à trois alcanes, le pentane, l'heptane et l'octane. Les résultats sont illustrés en figure 10E.

Lors de l'exposition à des alcanes, des différences fractionnelles de résistance négatives ont été observées.

Cette couche sensible a ensuite été exposée à trois alcools primaires avec différents encombrements. Les résultats sont illustrés en figure 10F.

On peut conclure des figures 10C à 10F que lorsqu'un alcool primaire est encombré, sa différence fractionnelle de résistance diminue par rapport à l'alcool non encombré.

Les polymères conducteurs permettent de passer outre les limites (modularité, spécificité aux composés polaires, consommation énergétique...) des oxydes métalliques ou des matériaux composites. Leurs compositions chimiques sont semblables à celles des COV, ce qui induit des interactions physiques entre le polymère et le COV. Par ailleurs, leurs structures sont modifiables, ce qui permet de créer des matériaux avec des sélectivités définies afin de cibler les COV.

EXEMPLE 7 : INTEGRATION ET PILOTAGE DU SYSTEME

8. INTERFACE DE COMMANDE DU SYSTEME D'ANALYSE

8.1 ARCHITECTURE DU SYSTEME D'ANALYSE

8.1.A Principe du système d'analyse

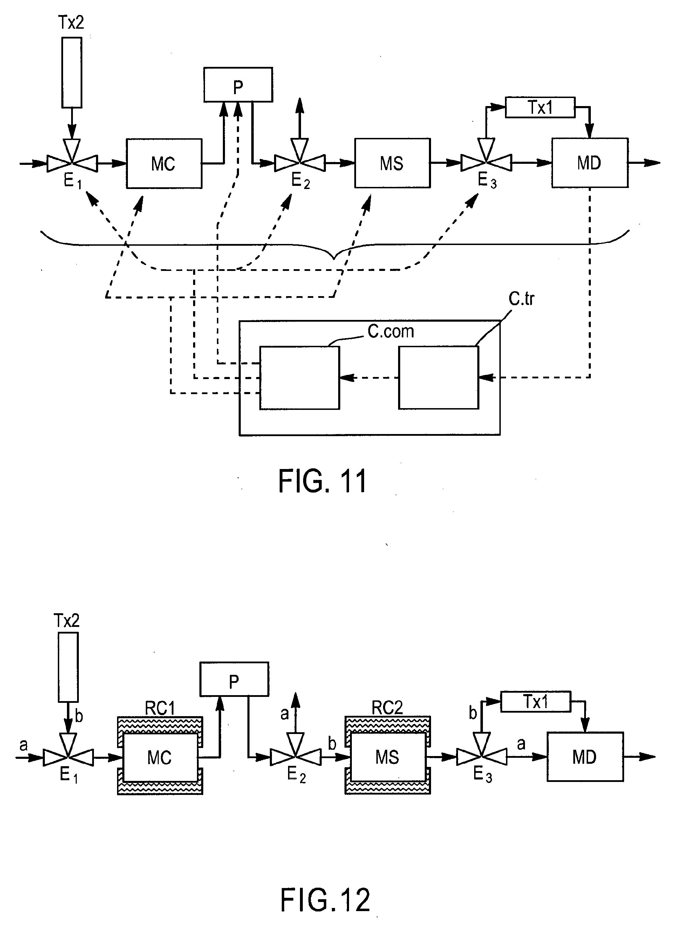

Le schéma de principe du système est présenté en Figure 11 et se compose donc des trois modules (le module de préconcentration, le module de séparation et le module de détection), d'une pompe P et de 3 électrovannes E1, E2, E3.

La pompe P sélectionnée est par exemple une pompe excentrique à membrane, commercialisée par Scharzer Precision sous la référence SP 725 EC. La pompe fonctionne sous alimentation continue entre 0 et 24 V. Les caractéristiques de la pompe ont été choisies en fonction de la perte de charge engendrée par l'utilisation de microcanaux comprise entre 1 et 2 bars.

Durant la phase de concentration, la pompe permet le prélèvement de l'échantillon par une circulation d'air à travers le module. Ainsi, par procédé d'accumulation des molécules, après la concentration, des molécules contenues dans l'échantillon d'air à analyser sont retenues dans la microstructure. Durant la phase d'analyse, en entrée du système, un filtre (charbon actif) permet par association avec la pompe, une circulation d'air propre à travers le système servant ainsi de gaz vecteur.

Les électrovannes permettent de choisir la direction des flux d'air durant les étapes de l'analyse. Des éléments chauffants associés à un régulateur de température sont également intégrés au système afin de permettre la chauffe des microstructures de concentration et de séparation pour permettre la libération des molécules piégées.

Les électrovannes utilisées dans le système sont des vannes électromagnétiques miniatures développées par Lee Company.

Concernant les éléments chauffants utilisés pour les modules de concentration et de séparation, le choix s'est porté sur des réchauffeurs RC en mica fabriqués par la société MINCO.

A titre illustratif, les caractéristiques des éléments utilisés pour chacune des deux microstructures sont reportées dans le tableau 11.

## Tableau 11 : Caractéristiques des réchauffeurs mica

| Réchauffeur concentrateur | | | |
|---|---|---|---|
| Dimensions (mm) | épaisseur (mm) | Résistance (Ohm) | surface effective (cm$^2$) |
| 25.4 x 101.6 | 0.5 | 21.2 | 16.13 |

| Réchauffeur colonne | | | |
|---|---|---|---|
| Dimensions (mm) | épaisseur (mm) | Résistance (Ohm) | surface effective (cm$^2$) |
| 50.8 x 50.8 | 0.5 | 23.2 | 18.06 |

Le système est piloté à l'aide d'une carte de commande -C Com- permettant, d'une part de gérer les commandes des électrovannes, des régulateurs de température et de la pompe, d'autre part, de récupérer les informations transmises par les capteurs par l'intermédiaire d'une carte de traitement -C Tr-.

8.1.B Description des étapes d'analyse

L'analyse d'un échantillon se décompose donc en deux étapes principales identiques à un système de chromatographie gazeuse classique, à savoir la concentration de l'échantillon puis l'analyse par séparation. L'utilisation des pompes et des électrovannes permet de diriger le flux d'air à travers les modules par des basculements de leurs états. Une illustration préférée du procédé présenté en Figure 12 associe les états possibles des différents éléments composant le système en fonction de l'étape d'analyse effectuée. Quatre situations représentatives de l'étape de l'analyse peuvent ainsi être définies : « inactif » 10, « concentration » 20, « analyse molécule » 40 et « nettoyage capteurs » 30.

Un état « inactif » 10 du système correspond à l'état du système au début et à la fin des étapes d'analyses.

Un état « concentration » 20 correspond à la première étape consistant à prélever l'air à analyser à travers la microstructure de concentration permettant ainsi la concentration de l'échantillon.

Les états « analyse molécule » 40 et « nettoyage capteurs » 30 correspondent aux deux états pris par le système en fonction de la présence ou de l'absence d'une molécule d'intérêt.

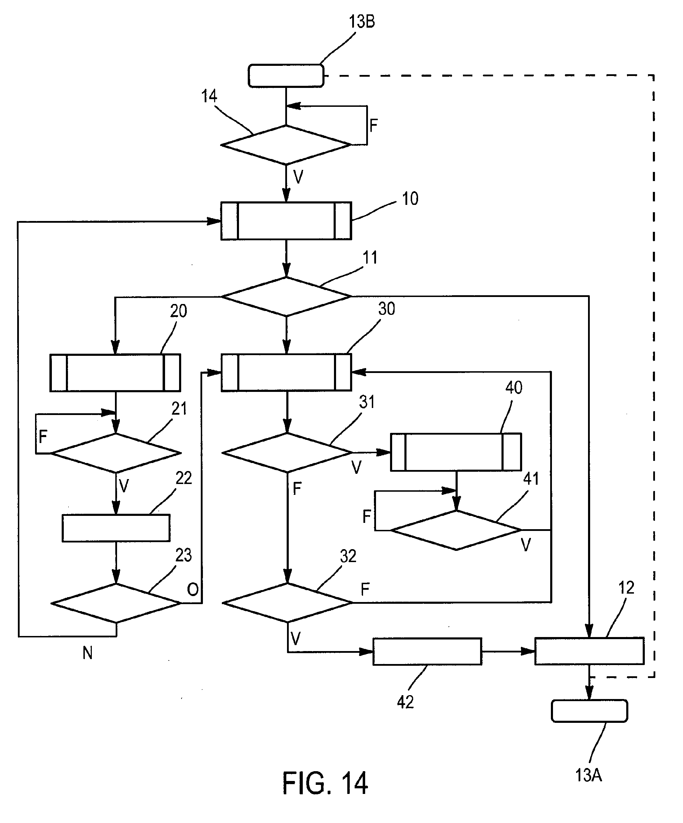

Les autres références de la figure 14 sont les suivantes :

| | | |
|---|---|---|
| V : Vrai; | F : Faux ; | O : Oui; |
| N : Non; | 13B : Début; | 14 : Init ?; |
| 11 : Choix étape?; | 21 : t=$t_{concen}$? | 23 : Analyse? |
| 31 : t=$t_{molécul}$? | 32 : t=$t_{analyse}$? | 41 : t=$t_{exno}$? |
| 42 : analyse terminée ; | 12 : arrêt système ; | 13A : Fin. |

Le système permet la séparation des molécules de l'échantillon avec un temps de rétention propre aux différentes molécules. En sortie du module de séparation, lorsque le temps d'analyse correspond au temps de rétention d'une des molécules d'intérêt, le système bascule dans l'état « analyse molécule » pendant quelques secondes afin de diriger les molécules vers le module de détection. En dehors du temps de passage d'une molécule à analyser, le système est maintenu à l'état « nettoyage capteurs » permettant ainsi une circulation d'air propre (filtré par charbon actif) dans le module de détection.

Le tableau 12 définit les états des différents éléments du système en fonction de l'étape d'analyse effectuée.

L'état de la pompe (P) est représenté par un état « On » et un état « Off » lorsqu'elles sont respectivement en état de marche ou éteinte. Les résistances chauffantes, nommées RC1 pour le module de concentration et RC2 pour le module de séparation, sont également représentées par un état « On » et un état « Off » lorsqu'elles sont respectivement alimentées ou non. Enfin, les électrovannes (numérotée de 1 à 3 sur la Figure 10) sont représentées par un état « a » ou un état « b » en fonction de la direction du flux d'air choisie.

Tableau 12 : Etats des éléments du système en fonction de l'étape d'analyse

| Etat du système | Vanne | | | Pompe | Resistance chauffantes | |
|---|---|---|---|---|---|---|
| | n°1 | n°2 | n°3 | | RC1 | RC2 |
| Inactif | b | b | b | Off | Off | Off |
| Concentration | a | a | b | On | Off | Off |
| Nettoyage capteurs | b | b | b | On | On | On |
| Analyse molécule | b | b | a | On | On | On |

8.1.C Description des éléments constitutifs du prototype

Le système peut utiliser des micro-éléments tels que les pompes P, les résistances chauffantes RC et les électrovannes E. Souhaitant obtenir un système le plus compact possible, ces éléments ont été sélectionnés avec un encombrement minimal en fonction de leurs performances pour répondre aux contraintes imposées par le système. Ces éléments sont disponibles commercialement chez divers spécialistes.

Ces éléments se composent d'un élément laminaire gravé pris entre deux couches de mica pour une épaisseur de 0,5 mm. Ces films peuvent atteindre rapidement des températures importantes (jusqu'à 600°C) avec une température homogène sur la surface du film. Les microstructures à chauffer sont de préférence mises en contact avec le réchauffeur entre deux couches d'isolant (papier céramique de 3,2 mm d'épaisseur) et maintenue mécaniquement entre deux plaques d'aluminium.

Un système de régulation en température permet de contrôler la température appliquée sur les microstructures. Ces vannes trois voies fonctionnent en utilisant un solénoïde permettant un verrouillage magnétique de la vanne vers un des deux ports d'entrée/sortie. Les dimensions de ces électrovannes sont conçues pour obtenir un faible volume interne (72 $\mu$L) afin de limiter les volumes morts.

8.2 OUTILS DE PILOTAGE DU SYSTEME

8.2.A Génération de signaux de commande

Comme on peut le voir sur la figure 13, le fonctionnement du dispositif est géré par une carte de commande C. Com. associée à une carte de traitement par l'intermédiaire d'un noeud de mesure No.

**[0292]** La carte de commande reçoit des informations des capteurs et les transmet par l'intermédiaire d'un montage en pont de Wheatstone Pwh associé à un montage amplificateur Ampli prévus ici dans la carte de traitement.

Une carte de démultiplexage Dmx est prévue dans la carte de commande, associée à des relais de commande des électrovannes (com EV), des résistances chauffantes (com RC), et d'un pont en H de commande de la pompe (H). Cette carte Dmx permet de générer différents signaux de commande à partir des niveaux logiques appliqués sur chacune des entrées numériques du noeud de mesure. La carte de commande comprend de préférence un convertisseur de tension conv entre le noeud No et la carte Dmx.

Une telle configuration permet une gestion de l'état des différents éléments du système afin de pouvoir les contrôler via un ordinateur.

Le noeud de mesure permet de transmettre les informations d'état du système envoyées par l'utilisateur. Le noeud de mesure offre ici 4 entrées analogiques 4A $\pm$10 V et 4 entrées numériques 4N bidirectionnelles. La carte de commande est donc conçue de façon à pouvoir générer différents états du système à l'aide des 4 bits d'entrées numériques 4N (DIO 0 à DIO 3) du noeud de mesure configurées en mode écriture. Les informations transmises par les capteurs du module de détection sont acheminées vers l'utilisateur en utilisant les 4 entrées analogiques du noeud de mesure.

Le schéma présenté en Figure 13 représente la partie génération des signaux de commande utilisée pour la conception de la carte de commande.

Les signaux de commande génèrent donc des tensions qui permettent de valider l'application d'une tension de commande des différents éléments par l'utilisation de relais -com-. Ces composants fonctionnent donc comme des interrupteurs commandés par les signaux de la carte de démultiplexage.

Les électrovannes étant commandées par la polarité de la tension appliquée, deux relais sont préférentiellement utilisés afin de les commander, un relais pour générer une polarité positive, l'autre pour générer une polarité négative. Lors du décodage par la carte Dmx, l'utilisation de 2 signaux de commande complémentaires est donc préférée pour chacune des électrovannes (soit 8 signaux).

Le pont en H -H com P- est préféré pour la commande des pompes de sorte à pouvoir utiliser des courants plus importants que pour les électrovannes.

8.2.C Conception de la carte

L'alimentation et la régulation de la tension des éléments composants la carte est faite de manière classique. Cet aspect est illustré par la référence Alim.

La carte de commande développée est ici une carte double face regroupant les différentes parties développées précédemment.

A partir des 4 entrées numériques du noeud de mesure (bus de commande), la carte Dmx permet donc de générer jusqu'à 16 signaux de commande (bus de donnée) après démultiplexage. Ceci permet ainsi de commander jusqu'à 4 pompes, 4 électrovannes et 2 résistances chauffantes. Une table de décodage peut ainsi être programmée dans la carte Dmx en fonction des différentes valeurs pouvant être prises par le bus de commande.

La table de décodage se compose donc simplement du bus de commande de 4 bits (DIO 0, DIO 1, DIO2 et DIO 3) et du bus de données composé par exemple de 8 signaux pour la commande de 4 électrovannes (6 signaux utilisés pour 3 électrovannes), 6 signaux pour la commande de 4 pompes (2 signaux utilisés pour 1 pompe) et 2 signaux pour la commande de deux résistances chauffantes (Figure 11).

8.3 INTERFACE UTILISATEUR

La programmation de la carte de démultiplexage permet de définir le cadencement de l'analyse par un changement de l'état du système en agissant sur la valeur du bus de commande composé des 4 entrées numériques du système et de permettre une visualisation graphique de l'état des différents éléments du système, le comportement physique étant géré par la carte de commande.

L'organigramme s'organise autour des 4 états possibles du système (« inactif » 10, « concentration » 20, « analyse molécule » 40 et « nettoyage capteurs » 30) décrits précédemment et générés par les deux bits d'entrée numériques DIO 0 et DIO 1. Dans les « sous-programmes » correspondant à chacun de ces états, deux actions sont effectuées : l'état des entrées numériques du bus de commande est transmis au noeud de mesure et les valeurs correspondantes à l'état des éléments du système sont affectées aux objets graphiques les représentant. Le procédé et l'organigramme sont davantage détaillés en partie 8.3.B ci-dessous.

Les entrées numériques DIO 2 et DIO 3 n'étant pas utilisées pour définir un état du système, celles-ci permettent de contrôler la régulation en température de la micro-colonne et la mise au repos des signaux de commande des électrovannes.

Les résistances chauffantes sont utilisées lors de la phase d'analyse afin de libérer les molécules piégées dans le module de préconcentration et de réguler la température du module de séparation. Aussi, si la chauffe du module de préconcentration ne nécessite pas une régulation contrôlée, celle-ci devant être réalisée le plus rapidement possible, la régulation en température du module de séparation peut influer sur le temps de rétention des molécules. L'entrée numérique DIO 2 n'étant pas utilisée pour définir une des phases d'analyse du système, celle-ci a été utilisée afin de contrôler la vitesse de chauffe du module de séparation en mode tout ou rien. Les changements d'états de l'entrée DIO 2, et donc de la commande de chauffe du module sont définis par un signal carré dont le rapport cyclique permet de créer virtuellement une rampe de température contrôlée.

La commande d'une électrovanne est réalisée par l'utilisation de deux relais complémentaires afin de pouvoir générer un état de commande selon le relais actionné. Une impulsion de commande est préférable pour faire basculer chacune des électrovannes en fonction de la polarité appliquée sur les entrées de commande.

Lorsque le système change d'état, un des relais est donc activé afin d'appliquer une polarité sur l'entrée de commande de chacune des électrovannes.

Ainsi, le tableau 13 présente la table de commande définie pour contrôler l'ensemble des éléments du système en fonction des étapes de l'analyse.

Tableau 13 : Table de commande du système

| Bus de commande | | | | Etat du système | Vanne | | | Pompe | Resistances Chauffantes | |
|---|---|---|---|---|---|---|---|---|---|---|
| DIO 0 | DIO 1 | DIO 2 | DIO 3 | | n°1 | n°2 | n°3 | | RC1 | RC2 |
| 0 | 0 | 0 | 0 | Inactif | -12V | -12V | -12V | Off | Off | Off |
| 1 | 0 | 0 | 0 | Concentration | +12V | +12V | -12V | On | Off | Off |
| 0 | 1 | 0 | 0 | Nettoyage capteurs | -12V | -12V | -12V | On | On | X |
| 1 | 1 | 0 | 0 | Analyse molécule | -12V | -12V | +12V | On | On | X |
| X | X | 0 | X | Commande de | X | X | X | X | X | Off |
| X | X | 1 | X | Chauffe RC2 | X | X | X | X | X | On |
| X | X | X | 0 | Repos Commande | +/-12V | +/-12V | +/-12V | X | X | X |
| X | X | X | 1 | électrovanne | 0V | 0V | 0V | X | X | X |
| * un X correspond à un état quelconque du signal | | | | | | | | | | |

### 8.3.B Conception de l'interface utilisateur

L'état des éléments du système (vannes, pompes et résistances chauffantes) en fonction des différentes étapes est répertorié dans le tableau 13. Les objets graphiques de visualisation de ces éléments sont donc affectés par des valeurs binaires permettant d'observer leurs évolutions au cours des différentes phases de l'analyse.

Les différents paramètres d'entrée du système retrouvés dans l'organigramme permettent de définir le cadencement des étapes de l'analyse.

Les deux premiers paramètres concernent donc le temps de prélèvement ($t_{concen}$) lors de la phase de concentration de l'échantillon et le temps nécessaire pour l'analyse de l'échantillon ($t_{analyse}$). Ces deux paramètres sont réglables par l'utilisateur à l'aide de boutons de contrôle sur l'interface utilisateur.

Le paramètre suivant concerne la gestion du système lors de l'analyse de l'échantillon. En effet lors de cette phase, le système peut se retrouver dans deux états, l'état de « nettoyage des capteurs » et l'état d'« analyse des molécules ». Lors de la phase d'initialisation, le système permet de définir le nombre de molécules qui devront être analysées en leurs associant leurs temps de rétention ($t_{molécul}$) et un temps d'exposition ($t_{expo}$), c'est-à-dire le temps où le système permet d'envoyer les molécules retenues vers le module de détection.

Ces données sont répertoriées dans un tableau et la comparaison entre le chronomètre associé au temps d'analyse de l'échantillon et les temps de rétention des molécules, permet de faire basculer le système dans l'état «analyse molécule» durant le temps d'exposition associé à la molécule.

### 9 CARACTERISATION DU SYSTEME D'ANALYSE

### 9.1 Caractérisation des débits

La pompe permet d'obtenir des débits relativement important lorsque celle-ci fonctionne à vide. Un palier induit par l'utilisation des microcanaux est observé pour une tension d'alimentation supérieure à 8 V. Le débit de prélèvement maximal est donc de 7 mL/min. La tension d'alimentation retenue est de 12 V permettant ainsi au système de conserver un débit de prélèvement identique en cas de pertes de charge supplémentaires engendrées par le prélèvement de particules par exemple.

### 9.2. Caractérisation des rampes de température

Une caractérisation des rampes de température est également réalisée pour chacune des microstructures de concentration et de séparation. La température maximale ajustée par le point de consigne des régulateurs est de 140°C.

Concernant la microstructure de concentration, cette température de consigne doit de préférence être obtenue le plus rapidement possible afin de libérer les molécules retenues dans un délai de temps le plus court possible. Une caractérisation des rampes de température obtenues pour différentes tension d'alimentions, et donc de puissance injectée à la résistance chauffante a donc été réalisée.

Afin d'obtenir la rampe de température la plus rapide possible dans ce premier prototype, la tension d'alimentation retenue est 20 V. La résistance chauffante utilisée pour la microstructure de concentration ayant une résistance nominale de 21,2 Ω, le courant consommé pour une tension de 20 V est d'environ 0,94 A, soit une puissance consommée de 18,8 W.

Le système a donc été conçu afin de pouvoir contrôler la vitesse de chauffe du module de séparation en créant une rampe de température par une alimentation en mode tout ou rien du régulateur.

Pour cela, une des entrées numériques (DIO 2) permet de contrôler directement le relais de commande de la résistance chauffante de la micro-colonne (RC2).

Les essais réalisés lors de la caractérisation de la micro-colonne ont montré qu'une température de 40°C suffit pour séparer les molécules d'intérêt. La valeur du rapport cyclique est donc fixée à 10 %.

Afin de permettre la libération des molécules nécessitant une température plus importante et donc permettre le nettoyage de la micro-colonne, celle-ci doit atteindre la température de consigne maximale.

La résistance chauffante utilisée pour la microstructure de séparation ayant une résistance nominale de 23,2 Ω, le courant consommé pour une tension de 20 V est d'environ 0,86 A, soit une puissance consommée de 17,2 W lorsque la commande de chauffe est activée.

De nombreuses combinaisons peuvent être envisagées sans sortir du cadre de l'invention ; l'homme de métier choisira l'une ou l'autre en fonction des contraintes économiques, ergonomiques, dimensionnelles ou autres qu'il devra respecter. Par exemple, l'homme du métier pourra configurer un microcontrôleur en lieu et place de la carte de commande et/ou la carte de traitement.

## Revendications

1. Dispositif de détection d'une contamination fongique dans un environnement intérieur comprenant :

   - un module de préconcentration (MC) ;
   - un module de séparation (MS) comprenant une micro-colonne chromatographique en aval du module de préconcentration (MC) ; et
   - un module de détection (MD) comprenant une matrice de capteurs en aval du module de séparation (MS),
   - des moyens de génération de flux de préférence au moins une pompe (P),

   le dispositif comprenant au moins une première électrovanne (E3) en amont du module de détection (MD) **caractérisé en ce que** la première électrovanne est placée entre le module de séparation (MS) et le module de détection (MD), permettant ainsi soit de diriger un flux qui comprend des molécules cibles vers le module de détection (MD), soit de diriger un flux filtré par un premier moyen de filtration (Tx1) permettant un nettoyage du module de détection (MD) lorsque le flux ne comprend pas les molécules cibles.

2. Dispositif de détection selon la revendication 1, **caractérisé en ce qu'**il est configuré de sorte qu'un même flux est soit dirigé vers le module de détection (MD) lorsqu'il comprend des molécules cibles, soit dirigé vers le premier moyen de filtration (Tx1) lorsqu'il ne comprend pas les molécules cibles.

3. Dispositif de détection selon la revendication 1 ou 2, **caractérisé en ce qu'**il comprend en outre au moins une deuxième électrovanne (E2) en amont du module de séparation permettant de diriger un flux soit vers le module de séparation (MS) lorsque le flux comprend des molécules cibles ou lorsque le flux est filtré par un moyen de filtration (Tx2), soit vers l'extérieur lorsque le flux ne comprend pas les molécules cibles.

4. Dispositif de détection selon la revendication 3 **caractérisé en ce que** ladite deuxième électrovanne est placée entre le module de concentration (MC) et le module de séparation (MS).

5. Dispositif de détection selon l'une des revendications 1 à 4, **caractérisé en ce qu'**il comprend en outre au moins une troisième électrovanne (E1) en amont du module de concentration (MC) permettant soit de diriger un flux de prélèvement d'échantillon vers le module de concentration (MC), soit de diriger un flux filtré par un moyen de filtration (Tx2) permettant un nettoyage du module de concentration lorsque le flux ne comprend pas les molécules cibles.

6. Dispositif de détection selon l'une des revendications 1 à 5, **caractérisé en ce que** l'un au moins des moyens de filtration comprend un polymère adsorbant de préférence à base de 2,6-diphénylène.

7. Dispositif de détection selon l'une des revendications 1 à 6, **caractérisé en ce que** les modules de concentration (MC) et/ou de séparation (MS) comprennent un matériau susceptible d'adsorber lesdites molécules cibles associé à un moyen de désorption correspondant.

**8.** Dispositif de détection selon la revendication 7, **caractérisé en ce que** le matériau susceptible d'adsorber lesdites molécules cibles est un polymère adsorbant de préférence des billes de polymère de préférence à base de 2,6 diphénylène oxyde, dans le cas du module de concentration et du diméthylpolysiloxane (PDMS) dans le cas du module de séparation, et le moyen de désorption comprend des résistances chauffantes prévues sur lesdits modules de concentration et/ou de séparation.

**9.** Dispositif de détection selon l'une des revendications 1 à 8, **caractérisé en ce qu'**il comprend en outre une interface de commande comprenant une carte de commande (C. Com) permettant de commander de préférence automatiquement, au moins l'une parmi lesdites électrovannes, un moyen d'élution en particulier des résistances chauffantes, un moyen de génération de flux en particulier la pompe.

**10.** Dispositif de détection selon la revendication 9, **caractérisé en ce que** l'interface de commande est connectée au module de détection de préférence par l'intermédiaire d'une carte de traitement (C. Tr), de sorte à recevoir des données de celui-ci.

**11.** Dispositif de détection selon la revendication 10, **caractérisé en ce que** la carte de traitement et le module de détection sont configurés pour mesurer une différence de résistivité entre le flux qui comprend les molécules cibles et le flux filtré.

**12.** Dispositif de détection selon l'une des revendications 1 à 11, **caractérisé en ce que** le module de détection comprend un polymère conducteur sélectionné dans le groupe comprenant le PEDOT-PSS, le bifluorène dibromé, le polypyrolle dopé avec de l'octane sulfonate, le polypyrrole dopé avec du perchlorate de lithium ou tout autre dérivé du polypyrrole, du polythiophène ou de la polyaniline.

**13.** Dispositif de détection selon l'une des revendications 9 à 12, **caractérisé en ce que** ladite interface de commande comprenant une carte de commande configurée pour commander de préférence automatiquement, lesdites électrovannes (E1,E2,E3), de sorte à réaliser au moins l'un parmi :

- soit diriger un flux qui comprend des molécules cibles vers le module de détection, soit de diriger un flux filtré par un premier moyen de filtration permettant un nettoyage du module de détection lorsque le flux ne comprend pas les molécules cibles,
- diriger un flux soit vers le module de séparation lorsque le flux comprend des molécules cibles ou lorsque le flux est filtré par un deuxième moyen de filtration, soit vers l'extérieur lorsque le flux ne comprend pas les molécules cibles,
- soit de diriger un flux de prélèvement d'échantillon vers le module de concentration, soit de diriger un flux filtré par un troisième moyen de filtration permettant un nettoyage du module de concentration lorsque le flux ne comprend pas les molécules cibles.

**14.** Dispositif de détection selon la revendication 13, **caractérisé en ce que** ladite interface de commande comprenant une carte de commande configurée pour commander en outre de préférence automatiquement, les moyens de génération de flux en particulier la au moins une pompe.

**15.** Dispositif de détection selon la revendication 13 ou 14, **caractérisé en ce que** ladite interface de commande comprenant une carte de commande configurée pour commander en outre, de préférence automatiquement, les moyens d'élution en particulier les résistances chauffantes de sorte à désorber les molécules cibles.

**16.** Procédé de détection d'une contamination fongique dans un environnement intérieur à partir d'un dispositif de détection comprenant :

- un module de préconcentration ;
- un module de séparation comprenant une micro-colonne chromatographique en aval du module de préconcentration et
- un module de détection comprenant une matrice de capteurs en aval du module de séparation,
- des moyens de génération de flux de préférence une pompe,

le procédé comprenant des étapes de :

- concentration (20) dans laquelle des molécules cibles sont retenues dans le module de préconcentration de préférence pendant un temps de concentration,
- nettoyage de capteurs (30), dans laquelle un flux filtré passe à travers au moins l'un parmi le module de préconcentration, le module de séparation et le module de détection, des moyens de désorption,
- analyse (40), dans laquelle les molécules cibles passent dans le module de détection, de préférence pendant un temps d'analyse.

**17.** Procédé selon la revendication 16, **caractérisé en ce qu'**il comprend au moins une étape inactive (12,13,10) avant et/ou après lesdites étapes de concentration et d'analyse, dans laquelle au moins les moyens de génération de flux sont inactivés, les étapes du procédé étant de préférence mises en oeuvre en continu de sorte à détecter une contamination fongique dans un environnement intérieur.

**18.** Procédé selon la revendication 16 ou 17, **caractérisé en ce qu'**il comprend les étapes pour commander de préférence automatiquement, au moins une électrovanne, de sorte à réaliser au moins l'une parmi les actions de :

- soit diriger un flux qui comprend des molécules cibles vers le module de détection, soit de diriger un flux filtré par un premier moyen de filtration permettant un nettoyage du module de détection lorsque le flux ne comprend pas les molécules cibles,
- diriger un flux soit vers le module de séparation lorsque le flux comprend des molécules cibles ou lorsque le flux est filtré par un deuxième moyen de filtration, soit vers l'extérieur lorsque le flux ne comprend pas les molécules cibles,
- soit de diriger un flux de prélèvement d'échantillon vers le module de concentration, soit de diriger un flux filtré par un troisième moyen de filtration permettant un nettoyage du module de concentration lorsque le flux ne comprend pas les molécules cibles.

**19.** Procédé selon l'une des revendications 16 à 18, **caractérisé en ce qu'**il comprend en outre des étapes pour commander de préférence automatiquement, le moyen de génération de flux en particulier la pompe de sorte à réaliser lesdites direction de flux.

**20.** Procédé selon l'une des revendications 16 à 19, **caractérisé en ce qu'**il comprend en outre des étapes pour commander de préférence automatiquement, les moyens d'élution en particulier les résistances chauffantes de sorte à désorber les molécules cibles.

**21.** Produit de programme informatique chargeable dans la mémoire d'une unité de commande comprenant des parties de code logiciel pour réaliser le procédé selon l'une des revendications 16 à 20 lorsqu'il est mis en oeuvre par une unité de commande.

**22.** Dispositif de détection selon l'une des revendications 13 à 15 comprenant un produit de programme informatique selon la revendication 21dans ladite interface de commande.

**Patentansprüche**

**1.** Vorrichtung zum Erkennen einer Pilzbelastung in einem Innenraum, umfassend:

- ein Vorkonzentrationsmodul (MC),
- ein Trennmodul (MS), umfassend eine chromatographische Mikrosäule, nachgelagert vom Vorkonzentrationsmodul (MC) und
- ein Erkennungsmodul (MD), umfassend eine Sensoranordnung nachgelagert vom Trennmodul (MS),
- Mittel zur Erzeugung eines Stroms, vorzugsweise mindestens eine Pumpe (P), wobei die Vorrichtung mindestens ein erstes Elektroventil (E3) vorgelagert vom Erkennungsmodul (MD) umfasst, **dadurch gekennzeichnet, dass** das erste Elektroventil zwischen dem Trennmodul (MS) und dem Erkennungsmodul (MD) angeordnet ist, wodurch ermöglicht wird, einen Strom, der Zielmoleküle umfasst, entweder zum Erkennungsmodul (MD) zu lenken, oder einen Strom, der von einem ersten Filtermittel (Tx1) gefiltert ist, zu lenken, der eine Reinigung des Erkennungsmoduls (MD) ermöglicht, wenn der Strom die Zielmoleküle nicht umfasst.

**2.** Vorrichtung zum Erkennen nach Anspruch 1, **dadurch gekennzeichnet, dass** sie derart konfiguriert ist, dass ein gleicher Strom entweder zum Erkennungsmodul (MD) gelenkt wird, wenn er Zielmoleküle umfasst, oder zum ersten Filtermittel (Tx1) gelenkt wird, wenn er die Zielmoleküle nicht umfasst.

**3.** Vorrichtung zum Erkennen nach Anspruch 1 oder 2, **dadurch gekennzeichnet, dass** sie außerdem mindestens ein zweites Elektroventil (E2) vorgelagert vom Trennmodul umfasst, das ermöglicht, einen Strom entweder zum Trennmodul (MS) zu lenken, wenn der Strom Zielmoleküle umfasst, oder wenn der Strom von einem Filtermittel (Tx2) gefiltert ist, oder nach Außen, wenn der Fluss die Zielmoleküle nicht umfasst.

**4.** Vorrichtung zum Erkennen nach Anspruch 3, **dadurch gekennzeichnet, dass** das zweite Elektroventil zwischen dem Konzentrationsmodul (MC) und dem Trennmodul (MS) angeordnet ist.

**5.** Vorrichtung zum Erkennen nach einem der Ansprüche 1 bis 4, **dadurch gekennzeichnet, dass** sie außerdem mindestens ein drittes Elektroventil (E1) vorgelagert vom Konzentrationsmodul (MC) umfasst, das ermöglicht, entweder einen Strom zur Probeentnahme zum Konzentrationsmodul (MC) zu lenken, oder einen Strom, der von einem Filtermittel (Tx2) gefiltert ist, zu lenken, der eine Reinigung des Konzentrationsmoduls ermöglicht, wenn der Strom die Zielmoleküle nicht umfasst.

**6.** Vorrichtung zum Erkennen nach einem der Ansprüche 1 bis 5, **dadurch gekennzeichnet, dass** mindestens eines der Filtermittel ein adsorbierendes Polymer umfasst, vorzugsweise auf der Grundlage von 2,6-Diphenylen.

**7.** Vorrichtung zum Erkennen nach einem der Ansprüche 1 bis 6, **dadurch gekennzeichnet, dass** das Konzentrations- (MC) und/oder Trennmodul (MS) ein Material umfasst, das die Zielmoleküle absorbieren kann, die mit einem entsprechenden Desorptionsmittel assoziiert sind, adsorbieren kann.

**8.** Vorrichtung zum Erkennen nach Anspruch 7, **dadurch gekennzeichnet, dass** das Material, das die Zielmoleküle adsorbieren kann, ein Polymer ist, das vorzugsweise Polymerkügelchen adsorbiert, vorzugsweise auf der Grundlage von 2,6 Diphenylenoxyd, im Fall des Konzentrationsmoduls, und Dimethylpolysiloxan (PDMS) im Fall des Trennmoduls, und das Desorptionsmittel Heizwiderstände umfasst, die auf den Konzentrations- und/oder Trennmodulen vorgesehen sind.

**9.** Vorrichtung zum Erkennen nach einem der Ansprüche 1 bis 8, **dadurch gekennzeichnet, dass** sie außerdem eine Steuerschnittstelle umfasst, die eine Steuerkarte (C. Com) aufweist, die es ermöglicht, vorzugsweise automatisch mindestens eines von den Elektroventilen, einem Elutionsmittel, insbesondere Heizwiderständen, und Mitteln zur Erzeugung eines Stroms, insbesondere der Pumpe, zu steuern.

**10.** Vorrichtung zum Erkennen nach Anspruch 9, **dadurch gekennzeichnet, dass** die Steuerschnittstelle mit dem Erkennungsmodul verbunden ist, vorzugsweise mit Hilfe einer Verarbeitungskarte (C. Tr), um Daten von dieser zu erhalten.

**11.** Vorrichtung zum Erkennen nach Anspruch 10, **dadurch gekennzeichnet, dass** die Verarbeitungskarte und das Erkennungsmodul konfiguriert sind, um einen Widerstandsunterschied zwischen dem Strom, der die Zielmoleküle umfasst, und dem gefilterten Strom zu messen.

**12.** Vorrichtung zum Erkennen nach einem der Ansprüche 1 bis 11, **dadurch gekennzeichnet, dass** das Erkennungsmodul ein leitfähiges Polymer umfasst, ausgewählt aus der Gruppe, bestehend aus PEDOT-PSS, dibromiertem Bifluoren, Polypyrrol, dotiert mit Octansulfonat, Polypyrrol, dotiert mit Lithiumperchlorat oder jedem anderen Derivat von Polypyrrol, Polythiophen oder Polyanilin.

**13.** Vorrichtung zum Erkennen nach einem der Ansprüche 9 bis 12, **dadurch gekennzeichnet, dass** die Steuerschnittstelle eine Steuerkarte umfasst, die konfiguriert ist, um vorzugsweise automatisch die Elektroventile (E1, E2, E3) zu steuern, um mindestens eines der Folgenden durchzuführen:

- entweder einen Strom, der Zielmoleküle umfasst, zum Erkennungsmodul zu lenken, oder einen Strom, der von einem ersten Filtermittel gefiltert ist, zu lenken, der eine Reinigung des Erkennungsmoduls ermöglicht, wenn der Fluss die Zielmoleküle nicht umfasst,
- einen Strom entweder zum Trennmodul zu lenken, wenn der Strom Zielmoleküle umfasst, oder wenn der Fluss von einem zweiten Filtermittel gefiltert ist, nach Außen, wenn der Strom die Zielmoleküle nicht umfasst,
- entweder einen Strom zur Probeentnahme zum Konzentrationsmodul zu lenken, oder einen Strom, der von einem dritten Filtermittel gefiltert ist, zu lenken, der eine Reinigung des Konzentrationsmoduls ermöglicht, wenn der Fluss die Zielmoleküle nicht umfasst.

**14.** Vorrichtung nach Anspruch 13, **dadurch gekennzeichnet, dass** die Steuerschnittstelle eine Steuerkarte umfasst, die konfiguriert ist, um außerdem vorzugsweise automatisch die Mittel zur Erzeugung eines Stroms zu steuern, insbesondere die mindestens eine Pumpe.

**15.** Vorrichtung nach Anspruch 13 oder 14, **dadurch gekennzeichnet, dass** die Steuerschnittstelle eine Steuerkarte umfasst, die konfiguriert ist, um außerdem vorzugsweise automatisch die Elutionsmittel zu steuern, insbesondere die Heizwiderstände, um die Zielmoleküle zu desorbieren.

**16.** Verfahren zum Erkennen einer Pilzbelastung in einem Innenraum auf der Grundlage einer Vorrichtung zum Erkennen, umfassend:

- ein Vorkonzentrationsmodul,
- ein Trennmodul, das eine chromatographische Mikrosäule, nachgelagert vom Vorkonzentrationsmodul umfasst, und
- ein Erkennungsmodul, das eine Sensoranordnung nachgelagert vom Trennmodul umfasst,
- Mittel zur Erzeugung eines Stroms, vorzugsweise eine Pumpe, wobei das Verfahren die Folgenden Schritte umfasst:
- Konzentrieren (20), wobei Zielmoleküle im Vorkonzentrationsmodul zurückgehalten werden, vorzugsweise während einer Konzentrationszeit,
- Reinigen der Sensoren (30), wobei ein gefilterter Strom durch mindestens eines des Vorkonzentrationsmoduls, des Trennmoduls und des Erkennungsmoduls Desorptionsmittel passieren lässt,
- Analysieren (40), wobei die Zielmoleküle in das Erkennungsmodul passieren, vorzugsweise während einer Analysezeit.

**17.** Verfahren nach Anspruch 16, **dadurch gekennzeichnet, dass** es mindestens einen inaktiven Schritt (12, 13, 10) vor und/oder nach den Schritten des Konzentrierens und des Analysierens umfasst, wobei mindestens die Mittel zur Erzeugung eines Stroms inaktiv sind, wobei die Schritte des Verfahrens vorzugsweise kontinuierlich durchgeführt werden, um eine Pilzbelastung in einem Innenraum zu erkennen.

**18.** Verfahren nach Anspruch 16 oder 17, **dadurch gekennzeichnet, dass** es die Schritte umfasst um vorzugsweise automatisch mindestens ein Elektroventil zu steuern, um mindestens einen der folgenden Vorgänge durchzuführen:

- entweder einen Strom, der Zielmoleküle umfasst, zum Erkennungsmodul zu lenken, oder einen Strom, der von einem ersten Filtermittel gefiltert ist, zu lenken, der eine Reinigung des Erkennungsmoduls ermöglicht, wenn der Strom die Zielmoleküle nicht umfasst,
- einen Strom entweder zum Trennmodul zu lenken, wenn der Strom Zielmoleküle umfasst, oder wenn der Strom von einem zweiten Filtermittel gefiltert ist, oder nach Außen, wenn der Strom die Zielmoleküle nicht umfasst,
- entweder einen Strom zur Probeentnahme zum Konzentrationsmodul zu lenken, oder einen Fluss, der von einem dritten Filtermittel gefiltert ist, zu lenken, der eine Reinigung des Konzentrationsmoduls ermöglicht, wenn der Strom die Zielmoleküle nicht umfasst.

**19.** Verfahren nach einem der Ansprüche 16 bis 18, **dadurch gekennzeichnet, dass** es außerdem Schritte umfasst um vorzugsweise automatisch das Mittel zum Erzeugen eines Stroms zu steuern, insbesondere die Pumpe, um die Lenkung des Stroms durchzuführen.

**20.** Verfahren nach einem der Ansprüche 16 bis 19, **dadurch gekennzeichnet, dass** es außerdem Schritte umfasst, um vorzugsweise automatisch die Elutionsmittel zu steuern, insbesondere die Heizwiderstände, um die Zielmoleküle zu desorbieren.

**21.** Computerprogrammprodukt, das in den Speicher einer Steuereinheit geladen werden kann, umfassend Softwarecode-Teile, um das Verfahren nach einem der Ansprüche 16 bis 20 durchzuführen, wenn es durch eine Steuereinheit ausgeführt wird.

**22.** Vorrichtung zum Erkennen nach einem der Ansprüche 13 bis 15, umfassend ein Computerprogrammprodukt nach Anspruch 21 in der Steuerschnittstelle.

**Claims**

1. Detection device of fungal contamination in an interior environment, including:

   - a preconcentration module (MC);
   - a separation module (MS) including a chromatographic microcolumn downstream of the preconcentration module (MC); and
   - a detection module (MD) including a sensor matrix downstream of the separation module (MS),
   - flow generating means, preferably at least one pump (P),

   The device includes at least one first solenoid valve (E3) upstream of the detection module (MD) **characterized in that** the first solenoid valve is placed between the separation module (MS) and the detection module (MD), enabling either to direct a flow containing target molecules toward the detection module (MD), or to direct a flow filtered by a first filtering means (Tx1) enabling the detection module (MD) to be cleaned when the flow does not contain the target molecules.

2. Detection device according to claim 1, **characterized in that** it is configured so that the same flow is directed to either the detection module (MD) when it comprises the target molecules, or directed towards the first filtering means (Tx1) when it does not include the target molecules.

3. Detection device according to claim 1 or 2, **characterized in that** it further comprises at least a second solenoid valve (E2) upstream of the separation module enabling to direct a flow to the separation module (MS) when the flow comprises target molecules or when the flow is filtered by a filtering means (Tx2), or toward the outside when the flow does not contain the target molecules.

4. Detection device according to claim 3, **characterized in that** said second solenoid valve is placed between the concentration module (MC) and the separation module (MS).

5. Detection device according to any of claims 1 to 4, **characterized in that** it also includes at least one third solenoid valve (E1) upstream of the concentration module (MC) enabling either to direct a sample collection flow toward the concentration module (MC), or to direct a flow filtered by a filtering means (Tx2), enabling the concentration module to be cleaned when the flow does not contain the target molecules.

6. Detection device according to any of claims 1 to 5, **characterized in that** at least one of the filtering means includes an adsorbent polymer, preferably based on 2,6-diphenylene.

7. Detection device according to any of claims 1 to 6, **characterized in that** the concentration (MC) and/or separation (MS) modules include a material capable of adsorbing said target molecules associated with corresponding desorption means.

8. Detection device according to claim 7, **characterized in that** the material capable of adsorbing said target molecules is an adsorbent polymer, preferably polymer beads, preferably based on 2,6-diphenylene oxide, in the case of the concentration module, and polydimethylsiloxane (PDMS) in the case of the separation module, and the desorption means include heating resistors provided on said concentration and/or separation modules.

9. Detection device according to any of claims 1 to 8, **characterized in that** it also includes a control interface including a control card (C. Com) enabling to control, preferably automatically, at least one among said solenoid valves, elution means, in particular heating resistors, and flow generating means, in particular the pump.

10. Detection device according to claim 9, **characterized in that** the control interface is connected to the detection module preferably by means of a processing card (C. Tr), so as to receive data from it.

11. Detection device according to claim 10, **characterized in that** the processing card and the detection module are configured so as to measure a difference in resistivity between the flow containing the target molecules and the filtered flow.

12. Detection device according to any of claims 1 to 11, **characterized in that** the detection module includes a conductive polymer selected from the group including PEDOT-PSS, dibromine bifluorene, polypyrrole doped with octane sulfonate, polypyrrole doped with lithium perchlorate or any other derivative of polypyrrole, polythiophene or polyaniline.

13. Detection device according to any of claims 9 to 12, **characterized in that** the said control interface including a control card configured so as to control, preferably automatically, said solenoid valves (E1, E2, E3), so as to perform at least one of the following:

- either direct a flow containing target molecules toward the detection module, or direct a flow filtered by the first filtering means enabling the detection module to be cleaned when the flow does not contain the target molecules,
- direct a flow either toward the separation module when the flow contains target molecules or when the flow is filtered by the second filtering means, or toward the outside when the flow does not include the target molecules,
- or direct a sample collection flow toward the concentration module, or direct a flow filtered by a third means for filtering enabling to clean the concentration module when the flow does not contain the target molecules.

14. Detection device according to claims 13, **characterized in that** the said control interface including a control card configured so as to also control, preferably automatically, the flow generating means, in particular the at least one pump.

15. Detection device according to claim 13 or, **characterized in that** the said control interface including a control card configured so as to also control, preferably automatically, the elution means, in particular the heating resistors so as to desorb the target molecules.

16. Process for detecting fungal contamination in an interior environment using a detection device including:

- a preconcentration module;
- a separation module including a chromatographic microcolumn downstream of the preconcentration module; and
- a detection module including a sensor matrix downstream of the separation module,
- flow generating means, preferably at least one pump,

the process including steps of:

- concentration (20), in which target molecules are retained in the preconcentration module, preferably for a concentration time;
- sensor cleaning (30), in which a filtered flow passes through at least one among the preconcentration module, the separation module or the detection module,
- analysis (40), in which the target molecules pass into the detection module, preferably for an analysis time.

17. Process according to claim 16, **characterized in that** it includes at least one inactive step (12, 13, 10) before and/or after said concentration and analysis steps, in which at least the flow generating means are inactivated, the steps of the process preferably being implemented continuously so as to detect fungal contamination in an interior environment.

18. Process according to claim 16 to 17, **characterized in that** it includes steps for controlling, preferably automatically, at least one solenoid valve, so as to perform at least one of the following actions:

- either direct a flow containing target molecules toward the detection module, or direct a flow filtered by a first filtering means enabling the detection module to be cleaned when the flow does not contain the target molecules,
- direct a flow either toward the separation module when the flow includes target molecules or when the flow is filtered by a second filtering means, or toward the outside when the flow does not include the target molecules,
- or direct a sample collection flow toward the concentration module, or direct a flow filtered by a third filtering means enabling the concentration module to be cleaned when the flow does not contain the target molecules.

19. Process according to any of claims 16 to 18, **characterized in that** it also includes steps for controlling, preferably automatically, the flow generating means, in particular the pump, so as to carry out said flow directions.

20. Process according to any of claims 16 to 19, **characterized in that** it also includes steps for controlling, preferably automatically, the elution means, in particular the heating resistors so as to desorb the target molecules.

**21.** Computer program capable of being loaded in the memory of a control unit including software code portions for performing the process according to any of claims 16 to 20 when it is implemented by a control unit.

**22.** Detection device according to any of claims 13 to 15 including a computer program according to claim 21 in the said control interface.

FIG. 1

FIG. 2

FIG. 3

FIG. 4

## FIG. 5

## FIG. 6

FIG. 7

FIG.8

FIG. 9

FIG.10A

FIG. 10B

FIG. 10C

FIG. 10D

FIG. 10E

FIG. 10F

FIG. 11

FIG.12

FIG. 13

FIG. 14

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2913501

- WO 1059636 A